# EUROPEAN PATENT SPECIFICATION

(11) **EP 1 100 311 B1**
(45) Date of publication and mention of the grant of the patent: **10.10.2007**
(21) Application number: 99939009.9
(22) Date of filing: 05.08.1999
(51) Int. Cl.: A01H 5/00, A01H 4/00, A01H 1/00, C12N 15/00, C12N 15/05, C12N 15/10

(54) **NOVEL GENETIC MATERIALS FOR TRANSMISSION INTO MAIZE**
NEUARTIGE, GENETISCHE MATERIALIEN ZUR ÜBERTRAGUNG IN DEN MAIS
NOUVELLES MATIERES GENETIQUES DESTINEES A ETRE TRANSMISES AU MAIS

(30) Priority: 05.08.1998 US 95400 P
(43) Date of publication of application: 23.05.2001
(73) Proprietor: Eubanks, Mary Wilkes, Durham, NC 27705 (US)
(72) Inventor: Eubanks, Mary Wilkes, Durham, NC 27705 (US)
(74) Representative: Bentham, Andrew
(86) International application number: PCT/US1999/017716
(87) International publication number: WO 2000/007434

(56) References cited:
- US-A- 5 330 547
- US-A- 5 710 367
- US-A- 5 750 828
- CHEMICAL ABSTRACTS, vol. 124, no. 1, 1 January 1996 (1996-01-01) Columbus, Ohio, US; abstract no. 5068, TSANEV, V. ET AL: "Use of electrophoretic patterns of esterase and prolamins for proving genetic material from teosinte and Tripsacum in maize genome" XP002228253 & DOKLADI NA BULGARSKATA AKADEMIYA NA NAUKITE ( 1994 ), 47(10), 89-92,
- DATABASE CABA [Online] BOROVSKII, M. I. ET AL: "Mutations in the progeny of hybrids of maize with Tripsacum and teosinte" retrieved from STN Database accession no. 78:87737 XP002228254 & OTDALEN. GIBRIDIZATSIYA RAST. V MOLDAVII, ( 1976 ) PP. 4-6. PUBLISHER: STIINCA. KISHINEV SECONDARY SOURCE: REFERATIVNYI ZHURNAL (1977) 4.55.57, Nauchno-proizvodstvennoe ob"edinenie Gibrid, Moldavian SSR.
- EUBANKS M W: "Molecular analysis of crosses between Tripsacum dactyloides and Zea diploperennis (Poaceae)." THEORETICAL AND APPLIED GENETICS, vol. 94, no. 6-7, 1997, pages 707-712, XP002228281 ISSN: 0040-5752
- KHOKHLOV S.S., "Hybridization of Maize with Tripsacum in Relation to the Problem of Experimental Induction of Apomixis in Maize", APOMIXIS AND BREEDING. Nauka Publishers, Moscow, 1970, translated from Russian by Amerind Publishing Co. Pvt. Ltd., New Delhi, pages 199-220, XP002925610

## Description

### FIELD OF THE INVENTION

This invention relates generally to the fields of molecular genetics and plant breeding. More particularly, it relates to a means of moving novel, stably inherited, variant forms of DNA into maize (*Zea mays* L.), also referred to as corn in the United States. These novel DNA sequences, derived from intergeneric hybridization between Eastern gamagrass (*Tripsacum dactyloides* L.) and perennial teosinte (*Zea diploperennis* Iltis, Doebley and Guzmán), provide unique markers for assisting selection of desirable traits in plant breeding programs, for detection of target DNA sequences in genetic analyses, and for the identification of new genes for corn improvement that may enhance resistance to insect pests and diseases, drought stress tolerance, cold tolerance, perennialism, grain yield, totipotency, apomixis, improved root systems, tolerance of water-logged soils, tolerance of high-aluminum and acidic soils, improved grain quality, enhanced forage quality, and adaptability to a CO2 enriched atmosphere.

### BACKGROUND OF THE INVENTION

Molecular Genetics. Genetics is the study of genes and heritable traits in biological organisms. In plant breeding, the goal of molecular genetics is to identify genes that confer desired traits to crop plants, and to use molecular markers (DNA signposts that are closely associated with specific genes) to identify individuals that carry the gene or genes of interest in plants (Morris 1998), to determine the DNA sequences and characterize gene expression and function. A genetic marker is a variant allele that is used to label a biological structure or process throughout the course of an experiment. Variants in DNA and proteins are used as markers in molecular genetics. Genetic analysis of molecular variants can identify a particular gene that is important for a biological process. Mutation is the process whereby nucleotide sequences and genes change from the reference form generally designated wild type to a different form, and mutants are the source of variant genotypes in genetic analysis that allow selection of new phenotypes (Griffiths et al. 1993). Mutations occur at the level of a specified nucleotide sequence, the gene (i.e. DNA sequence), or the chromosome (i.e. the hereditary package in which units of DNA containing specific nucleotide sequences and genes are supercoiled with proteins). In a genetic mutation, the nucleotides that comprise the wild type allele of a gene (i.e. reference form that exists at a particular locus) is altered. In chromosome mutations, segments of chromosomes, whole chromosomes, or entire sets of chromosomes change via inversion, translocation, fusions and deletions.

In general, mutations are very rare, and most newly formed mutations are deleterious. Data on mutation frequencies for seven genes in maize provides a baseline indicating the rarity of mutations in maize (Stadler 1951). Mutation frequency ranged from 0.000492% (i.e. 492 mutants out of a million gametes) in the red color(*R*) gene; 0.000106% (i.e. 106 out of a million) for the inhibitor of *R* (*I*) gene; 0.000011% (i.e. 11 out of a million) for the purple aleurone (*Pr*) gene; 0.0000024% (i.e. 2.4 out of a million) for the starchy (*Su*) gene; 0.0000022% (i.e. 2.2 out of a million) for the yellow color (*Y*) gene; 0.0000012% (i.e. 1.2 out of a million) for the normal kernel (Sh) gene, and 0% (i.e. 0 out of a million for the waxy gene (*Wx*)*.*

Because spontaneous mutations are rare, geneticists and plant breeders typically use mutagens (i.e. agents such as chemicals and radiation to increase the frequency of mutation rates) to obtain variant forms that can be used in genetic analysis and selection of new varieties. Another method of inducing mutagenesis in maize is transposon tagging whereby a maize line is crossed with a line containing one of the three systems of transposable elements found in maize. When a transposable element inserts into a gene, it causes a mutation. The reported mutation frequencies for transposable element mutator lines varies from 1 in a thousand to 1 in a million (Chomet 1994). To find a mutation using one of these mutagenic lines, a breeder must screen a minimum of 100,000 progeny.

Plant Breeding. Conventional plant breeding is the science that utilizes crosses between individuals with different genetic constitutions. The resulting recombination of genes between different lines, families, species, or genera produces new hybrids from which desirable traits are selected. Plant breeding is achieved by controlling reproduction. Since maize is a sexually reproducing plant, techniques for controlled pollination are frequently employed to obtain new hybrids. Controlling reproduction in maize involves continually repeating two basic procedures: (1) evaluating a series of genotypes, and (2) self-pollinating or crossing among the most superior plants to obtain the next generation of genotypes or progeny. Controlled pollinations in maize utilize two procedures: (1) detasseling, and (2) hand pollination.

Maize is a monoecious grass that has separate male and female flowers on the same plant. The male or staminate flowers produce pollen in the tassel at the apex of the maize stalk, and the female or pistillate flowers that produce the grain when pollinated are borne laterally in leaf axils tangential to the stalk. Pollination is accomplished by transfer of pollen from the tassel to silks which emerge from the axillary pistillate ears. Since maize is wind-pollinated, controlled pollination in which pollen collected from the tassel of one plant and transferred by hand to the silks of the same or another plant, is a technique used in maize breeding. The steps involved in making controlled crosses and self-pollinations in maize are standard practice (Neuffer 1982) and are as follows: (1) the ear emerging from the leaf shoot is covered with an ear shoot bag one or two days before the silks emerge to prevent contamination by stray pollen; (2) prior to making a pollination, the ear shoot bag is quickly removed and the silks cut with a knife to form a short brush, then the bag is immediately placed back over the ear; (3) also prior to making a pollination, the tassel is covered with a tassel bag to collect pollen; (3) on the day crosses are made, the tassel bag with the desired pollen is carried to the plant for crossing, the ear shoot bag is removed and the pollen dusted on the silk brush, the tassel bag is then fastened in place over the pollinated shoot to protect the developing ear.

*Zea diploperennis* (hereafter referred to as diploperennis), is a diploid perennial teosinte and a wild relative of maize endemic to the mountains of Jalisco, Mexico. Diploperennis is in the same genus as maize, has the same chromosome number (2n=20), and can hybridize naturally with it.

*Tripsacum* is a polyploid, rhizomatous perennial grass that is a more distant wild relative of maize and has a different chromosome number (x=18, 2n=36 or 2n=72). *Tripsacum* is not know to naturally form fertile hybrids with maize or the wild Zeas. The progeny of (maize X *Tripsacum)* obtained by artificial methods have ten maize chromosomes and either 18 or 36 *Tripsacum* chromosomes and are male sterile. Female fertility can be partially restored using special techniques that eliminate most of the *Tripsacum* chromosomes (Mangelsdorf 1974). Plants obtained by crossing *Tripsacum* and maize (*Zea mays* L.) employing *Tripsacum* as the pollen donor have unreduced gametes with a complete set of Zea chromosomes and a complete set of *Tripsacum* chromosomes. There is one report of a successful reciprocal cross in which *Tripsacum* was pollinated by maize that required embryo culture techniques to bring the embryo to maturity, and the plants were sterile (Farquharson 1957). Maize-Tripsacum hybrids have been crossed with teosinte to created a trigenomic hybrid that has a total of 38 chromosomes; 10 from maize, 18 from *Tripsacum* and 10 from teosinte. The resulting trigenomic plants were all male sterile and had a high degree of female infertility (Mangelsdorf 1974; Galinat 1986).

Based on known crossability relationships between Zea and *Tripsacum* and the results of prior crosses between them, the success of the crosses between Zea *diploperennis* and *Tripsacum* resulting in viable, fully fertile plants with chromosome numbers of 2n=20 (Eubanks 1995, 1997) could not have been predicted. Reduction in chromosome number in the interspecific crosses was unexpected based on prior art. The fertility of plants resulting from the cross made both ways with *Tripsacum* as pollen donor and pollen recipient was also unexpected based on prior art.

Although the base chromosome numbers of *Tripsacum* and Zea *diploperennis* are different, x=10 in Zea and x=18 in *Tripsacum,* their respective total chromosome lengths are almost equal. The total length of the 18 *Tripsacum dactyloides* chromosomes is 492. 5µ (Chandravadana et al. 1971), and the total length of the 10 Zea *diploperennis* chromosomes is 501. 64µ (Pasupuleti and Galinat 1982). It is not easy to obtain a hybrid plant when crossing *Tripsacum* and diploperennis. Hundreds of pollinations are required to obtain a viable seed, and approximately half of seedlings that germinate die soon after germination. However, as evidenced by cross fertility and chromosome number, when precise alignments occur between homologous regions of the chromosomes of *Tripsacum* and diploperennis there is a sufficient degree of pairing to occasionally enable the rare and unexpected success of this cross.

The unexpected fertility of *Tripsacum-*perennial teosinte hybrids, and their cross-fertility with maize, are of great value because they provide opportunity for directly crossing the recombined intergeneric germplasm with maize. In addition to providing a genetic bridge for importing *Tripsacum* genes into maize, *Tripsacum*-diploperennis hybrids provide a mechanism for importing any new *Tripsacum* genes not found in maize or the wild Zeas, and any de novo genetic material that arises from these wide species crosses into maize using traditional plant breeding techniques.

DNA fingerprinting has revealed that new *Tripsacum* alleles not found in maize or the wild *Zeas* and de novo sequences newly created via the wide cross are stably inherited in the progeny of succeeding generations and can be conferred to maize by crossing maize with *Tripsacum*-diploperennis containing de novo nucleotide sequences and alleles unique to *Tripsacum.* For purposes herein, de novo genetic material refers to regions where new allelic forms of DNA sequences are repeatedly and reliably created whenever crosses between *Tripsacum* and Zea *diploperennis* produce viable, fertile plants.

Feasibility has been demonstrated in plants derived from crossing *Tripsacum-*diploperennis with maize that exhibit resistance to western corn rootworm (*Diabrotica virgifera* Le Conte) and corn borer, tolerance to drought, and have properties of perennialism. Investigation and characterization of association with other traits such as response to high levels of atmospheric CO2 are in process.

### References Cited

U. S. PATENT DOCUMENTS
4,545,146 10/1985 Davis
4,627,192 12/1986 Fick
4,684,612 8/1989 Hemphill et al
4,737,596 4/1988 Seifert et al.
4,837,152 6/1989 Hemphill and Warshaw
5,059,745 10/1991 Foley
PP 6,906 7/1989 Eubanks
PP 7,977 9/1992 Eubanks
5,330,547 7/1994 Eubanks
PP 9,640 9/1996 Eubanks
5,750,828 5/1998 Eubanks

### OTHER PUBLICATIONS

Chaganti, R.S.K. 1965. Cytogenetic studies of maize-Tripsacum hybrids and their derivatives. Harvard Univ. Bussey Inst., Cambridge, MA.
Chandravadana, P., W.C. Galinat and B.G.S. Rao. 1971. A cytological study of Tripsacum dactyloides. J. Hered. 62:280-284.
Chomet, P.S. 1994. Transposon tagging with Mutator. In M. Freeling and V. Walbot (eds.), The Maize Handbook, Springer-Verlag, New York.
Cohen, J.I. and W.C. Galinat. 1984. Potential use of alien germplasm for maize improvement. Crop Sci. 24:1011-1015.
Curtis, H. and N.S. Barnes. 1989. Biology. Worth Publishers, Inc. New York, NY.
Eubanks, M.W. 1995. A cross between two maize relatives: Tripsacum dactyloides and Zea diploperennis (Poaceae). Econ. Bot. 49:172-182.
Eubanks, M.W. 1997. Molecular analysis of crosses between Tripsacum dactyloides and Zea diploperennis (Poaceae). Theor. Appl. Genet. 94:707-712.
Farquharson, L.I. 1957. Hybridization of Tripsacum and Zea. J. Heredity 48:295-299.
Galinat, W.C. 1974. Intergenomic mapping of maize, teosinte and Tripsacum. Evolution 27:644-655.
Galinat, W.C. 1977. The origin of corn. In G.F. Sprague (ed.). Corn and Corn Improvement. Amer. Soc. Agronomy, Madison, WI.
Galinat, W.C. 1982. Maize breeding and its raw material. In W.L. Sheridan (ed.) Maize for Biological Research. University Press, Grand Forks, North Dakota.
Galinat, W.C. 1986. The cytology of the trigenomic hybrid. Maize Genetics Newsletter 60:133.
Gardiner, J. E.H. Coe, S. Melia-Hancock, D.A. Hoisington and S. Chao. 1993. Development of a core RFLP map in maize using an immortalized F2 population. Genetics 134:917-930.
Griffiths, A.J.F., J.H. Miller, D.T. Susuki, R.C. Lewin, and W.M.Gelbart. 1993. An Introduction to Genetic Analysis, 5th edition. W.H. Freeman and Co., New York.
Helentjaris, T., M. Slocum, S. Wright, A. Schaefer and J. Nienhuis. 1986. Construction of genetic linkage maps in maize and tomato using restriction fragment length polymorphisms. Theor. Appl. Genet. 72:761-769.
Jeffreys, A.J., N.J. Royle, W. Wilson and Z. Wong. 1988. Spontaneous mutation rates to new length alleles at tandem-repetitive hypervariable loci in human DNA. Nature 332:278-281.
Kindiger, B. and J.B. Beckett. 1990. Cytological evidence supporting a procedure for directing and enhancing pairing between maize and Tripsacum. Genome 33:495-500.
Kresovich, S., W.F. Lamboy, A.K. Szewc-McFadden, J.R. McFerson, and P.L. Forsline. 1993. Molecular diagnostics and plant genetic resources conservation. Agbiotech News and Information 5(7):255-258.
Lewin, B. 1997. Genes V. Oxford University Press, Oxford, UK.
Maguire, M.P. 1961. Divergence in Tripsacum and Zea chromosomes. Evolution 15:393-400.
Maguire, M.P. 1963. Chromatid interchange in allodiploid maize-Tripsacum hybrids. Can. J. Genet. Cytol. 5:414-420.
Mangelsdorf, P.C. 1974. Corn: Its origin, evolution and improvement. Harvard Univ. Press, Cambridge, MA.
Melchinger, A.E., M.M. Messmer, M. Lee, W.L. Woodman, and.K.R. Lamkey. 1991. Diversity and relationships among U.S. maize inbreds revealed by restriction fragment length polymorphisms. Crop Science 31:669-678.
Messmer, M.M., A.E. Melchinger, R. Herrmann, and J. Boppenmaier. 1993. Relationships among early European maize inbreds: II. Comparison of pedigree and RFLP data. Crop Science 33:944-950.
Morris, M . L . , Ed . 1998. Maize Seed Industries in Developing Countries. Lynne Rienner Publishers., Inc., Boulder, CO.
Neuffer, M.G. 1982. Growin maize for genetic purposes. In W.L. Sheridan (ed.) Maize for Biological Research. University Press, Grand Forks, North Dakota.
Neuffer, M.G., E.H. Coe and S.R. Wessler. 1997. Mutants of Maize. Cold Spring Harbor Laboratory Press, New York.
Pasupuleti, C.V. and W.C. Galinat . 1982. Zea diploperennis I.- Its chromosomes and comparative cytology. Heredity 73:168-170.
Poehlman, J.M. 1986. Breeding Field Crops. 3rd ed. AVI Publ. Co., Inc . ; Westport, CT.
Reeves, R.G. and A.J. Bockholt. 1964. Modification and improvement of a maize inbred by crossing it with Tripsacum. Crop Sci. 4:7-10.
Smith, O.S. and J.S.C. Smith. 1992. Measurement of genetic diversity among maize hybrids; a comparison of isozymic, RFLP, pedigree, and heterosis data. Maydica 37:53-60.
Stadler, L.J. 1951. Spontaneous mutation in maize. Cold Spring Harbor Symp. Quant, Biol 16:49-63.
Tantravahi, 1968. Cytology and crossability relationships of Tripsacum. Harvard Univ. Bussey Inst., Cambridge, MA.

### SUMMARY OF THE INVENTION

According to the invention, there is provided a method of screening a plant to determine whether the plant is a cross between *Tripsacum* and perennial teosinte, said method comprising the following steps:
(a) crossing a *Tripsacum* female parent with a perennial teosinte pollen parent to produce seed, or crossing a perennial teosinte female parent with a *Tripsacum* pollen donor to produce seed; then
(b) harvesting the seed produced in (a); then
(c) growing plants from the seed harvested in (b); then
(d) isolating the total genomic DNA from the plants in (c); then
(e) digesting said genomic DNA with one to four of the restriction enzymes selected from the group consisting of *Eco*RI, *Eco*RV, *Hind*III and *Bam*HI; then
(f) probing said digested genomic DNA with one or more DNA markers selected from the group consisting of the maize nuclear DNA probes, maize mitochondrial DNA probes, and *Tripsacum* DNA probes recited below; and then
(g) determining the presence of one or more of the following restriction fragments of the following fragment sizes, wherein said restriction fragments are characterized by the following molecular marker-restriction enzyme associations and the associated fragment sizes selected from the group consisting of:
   BNL5.62. *Eco*RI*,* 10.3 kb; npi97, *Hind*III*,* 3.9 kb; UMC157. *Eco*RI, 6.5 kb and 3.3 kb; UMC157, *Hind*III*,* 5.5 kb; UMC157, *Bam*HI*,* 14.0 kb, 8.6 kb and 4.5 kb; UMC11, *Bam*HI*,* 7.0 kb; CSU3, *Bam*HI*,* 10.0 kb, 7.6 kb and 3.5 kb; UMC67, *Eco*RI, 19.2 kb; UMC67, *Bam*HI 13.4 kb and 1.6 kb; CSU92, *Bam*HI*,* 13.3 kb and 7.5 kb; asg62, *Bam*HI*,* 12.7 kb, 9.7 kb and 6.6 kb; UMC58, *Hind*III*,* 3.3 kb; CSUI64,*Eco*RI, 9.0 kb and 7.0 kb; UMC128, *Hind*III*,* 6.0 kb; UMC107, *Eco*RI*,* 6.3 kb and 5.3 kb; UMC107. *Hind*III*,* 19.2 kb; UMC140, *Eco*RI*,* 5.0 kb; UMC140, *Hind*III, 6.5 kb; UMC107, *Eco*RI*,* 6.3 kb; adh1, *Hind*III, 9.4 kb; adh1, *Bam*HI*,* 9.4 kb; UMC161, *Hind*III*,* 3.3 kb; BNL8.29, *Hind*III*,* 8.3 kb; UMC53, *Eco*RI*,* 9.4 kb, 3.8 kb and 3.0 kb; UMC53, *EcoRV,* 8.4 kb, 3.8 kb and 3.0 kb; UMC6, *Eco*RI*,* 3.8 kb; UMC6, *Hind*III 9.4 kb; UMC6, *Bam*HI*,* 13.2 kb, 12.7 kb, and 7.0 kb; UMC61, *Hind*III*,* 3.4 and 2.8 kb; UMC34, *Eco*RI*,* 7.5 kb and 5.4 kb; UMC34, *Hind*III*,* 8.8 kb, 6.5 kb and 5.8 kb; UMC135. *Hind*III*,* 11.6 kb and 10.8 kb; UMC131, *Eco*RI*,* 10.6 kb, 5.8 kb and 4.3 kb; UMC55, *Eco*RI*,* 3.9 kb; UMC55, *Hind*III*,* 4.3 kb; UMC5, *Eco*RI*,* 5.4 kb; UMC5, *Hind*III*,* 6.5 kb; UMC49, *Bam*HI*,* 8.2kb, 6.0 kb, 4.2 kb, and 3.2 kb; UMC36, *Bam*HI*,* 4.2 kb; UMC32, *Hind*III 6.7 kb; asg24, *Hind*III*,* 7.2 kb and 6.4 kb; UMC121, *Eco*RI*,* 3.7 kb and 3.2 kb; BNL8.35, *Hind*III*,* 9.9 kb and 8.7 kb; UMC50, *Bam*HI, 7.8 kb, 6.8 kb, 5.8 kb and 3.8 kb; UMC42, *Hind*III, 10.4 kb, 8.9 kb, 7.6 kb, 3.7 kb and 3.0 kb; npi247, *Eco*RI, 8.0 kb; npi247, *Hind*III 3.0 kb; UMC10, *Hind*III, 5.9 kb and 3.0 kb; UMC10, *Eco*RI, 6.5 kb and 5.5 kb; UMC102, *Eco*RI, 2.7 kb; BNL6.06, *Eco*RI, 6.8 kb; BNL5.37, *Hind*III, 10.3 kb, 5.8 kb and 3.5 kb; npi296, *Eco*RI, 7.9 kb; UMC3, *Eco*RI 2.5 kb and 2.0 kb; npi212, *Hind*III, 4.3 kb; npi212, *Bam*HI, 5.4 kb; UMC39, *Eco*RI, 12.2 kb, 9.2kb, 7.8 kb and 7.1 kb; UMC63, *Hind*III, 9.5 kb and 4.3kb; UMC96, *Hind*III, 11.8 kb, 6.4 kb and 5.5 kb; UMC96, *Bam*HI, 7.5 kb; UMC2, *Eco*RI, 11.8 kb, 10.4 kb and 8.0 kb; CSU25, *Hind*III, 4.5 and 4.3 kb; agrr115, *Eco*RI. 8.0 kb and 5.4 kb; agrr115, *Bam*HI, 5.4 kb and 3.5 kb; phi20725, *Eco*RI, 10.3 kb, 9.7 kb and 7.2 kb; phi20725, *Hind*III, 1.5 kb; UMC31, *Eco*RI, 5.8 kb; UMC31, *Bam*HI 6.5 kb; BNL5.46, *Hind*III*,* 13.7 kb, 10.5 kb, 9.7 kb and 5.1 kb; npi386, *Hind*III, 12.6 kb, 9.3 kb and 8.2 kb; tda62, *Bam*HI, 5.5 kb and 4.8 kb; BNL5.71, *Eco*RV, 11.3 kb, 6.8 kb, and 5.7 kb; BNL5.71. *Bam*HI, 11.3 kb, 6.8 kb and 5.7 kb; UMC156, *Hind*III, 3.0 kb; UMC66, *Eco*RI, 10.5 kb and 6.5 kb; UMC66, *Bam*HI, *3.7* kb; UMC19, *Bam*HI, 12.3 kb; UMC104, *Hind*III, 12.4 kb, 11.6 kb and 7.5 kb; UMC104, *Bam*HI, 9.4 kb; UMC133, *Hind*III, 10.6 kb, 9.9 kb, 9.2 kb and 7.7 kb; UMC52, *Bam*HI, 8.7 kb, 6.9 kb, 3.8 kb, 3.0 kb and 2.0 kb; BNL 15.07, *HindIII,* 2.9 kb and 2.7 kb; npi409, *Eco*RI, 9.4 kb; npi409, *Hind*III, 10.4 kb, 9.0 kb and 3.9 kb; UMC147, *Hind*III, 16.3 kb, 3.8 kb and 2.4 kb; UMC90, *Hind*III, 7.8 kb, 2.8 kb and 2.5 kb; UMC90, *Bam*HI, 9.0 kb; UMC27, *Hind*III, 8.3 and 4.5 kb; tda37, *Bam*HI, 8.2 kb and 6.5kb; UMC43. *Bam*HI*,* 9.7 kb, 7.3 kb and 5.7 kb; UMC40. *Bam*HI*,* 7.2 kb, 4.3 kb and 4.0 kb ; BNL7.71, *Hind*III, 10.6 kb; tda62. *Bam*HI*,* 5.5 kb; UMC68, *Hind*III*.* 6.0 kb; phi 10017. *Bam*HI*,* 15.1 kb and 9.5 kb; tda50. *Bam*HI*,* 8.5 kb; npi373, *Hind*III. 6.5 kb, 5.6 kb and 3.0 kb; tda204. *Bam*HI*,* 4.0 kb; npi393, *Eco*RI, 12.1 kb, 8.5 kb, 7.0 kb and 5.6 kb; UMC65. *Hind*III, 2.9 kb; UMC46. *Eco*RI, 6.5 kb and 5.6 kb; asg7, *Hind*III*,* 6.3 kb; UMC28, HindIII, 15.8 kb and 11.9 kb; UMC28, *Bam*HI, 10.0 kb, 7.6 kb and 6.6 kb; UMC134, *Bam*HI, 4.7 kb; UMC134, *Hind*III, 7.5 kb; asg8, *Hind*III, 10.8 kb, 8,7 kb and 8.4 kb; O2, *Eco*RI, 9.4 kb; BNL15.40, *Hind*III, 5.8 kb; UMC116, *Eco*RI, 9.5 kb; UMC110, *Bam*HI, 10.6 kb, 4.9 kb and 3.9 kb; BNL8.32. *Hind*III, 8.9 kb, 7.4 kb and 7.1 kb; BNL14.07, *Eco*RI, 6.4 kb; UMC80, *Hind*III, 10.6 kb, 8.2 kb and 2.4 kb; BNL16.06, *Eco*RI, 6.8 kb and 1.9 kb; phi20020, *Hind*III, 7.8 kb and 6.6 kb; npi114, *Hind*III, 10.0 kb, 8.8 kb and 6.3 kb; BNL9.11, *Hind*III, 3.4 kb; UMC103, *Hind*III, 6.9 kb and 5.7 kb; UMC124, *Hind*III, 8.0 and 7.0; UMC124, BamHI, 6.6 kb and 2.6 kb; UMC120, *Hind*III, 3.2 kb, 2.3 kb and 1.4 kb; UMC89, *Eco*RI, 7.3 kb; UMC89, *Hind*III, 7.3 kb; UMC89, *Bam*HI, 9.5 kb, 6.0 kb, 5.2 kb and 4.5 kb; BNL12.30, *Eco*RI, 3.5 kb; UMC48, *Hind*III, 6.2 kb, 5.3 kb, 4.7 kb, 3.5 kb and 2.2 kb; npi268, *Bam*HI, 6.4 kb; phi10005, *Eco*RI, 15.0 kb; UMC113, *Eco*RI, 5.9 kb and 5.4 kb; UMC113, *Bam*HI, 12.8 kb, 11.8 kb and 10.5 kb; UMC192, *Hind*III, 11.4 kb and 6.4 kb; wx (waxy), *Hind*III, 21.0 kb; CSU147, *Hind*III 5.9 kb; BNL5.10, *Hind*III*,* 6.1 kb and 4.4 kb; UMC114, *Bam*HI*,* 12.6 kb, 11.5 kb, 10.0 kb, 8.8 kb, 7.5 kb and 6.5 kb; UMC95 *Eco*RI*,* 5.6 kb; UMC95*, Hind*III*,* 7.7 kb, 4.8 kb and 4.1 kb; UMC95, *Bam*HI*,* 15.0 kb and 9.0 kb; CSU61. *Eco*RI*,* 8.1 kb and 4.8 kb; BNL7.57 *Eco*RI*,* 1.0 kb; BNL7.57, *Bam*HI*,* 11.6 kb, 5.9 kb and 1.3 kb; CSU54, *Eco*RI*,* 14.7 kb and 12.6 kb; phi20075, *Eco*RI, 7.1 kb; npi285, *Eco*RI, 12.4 kb, 9.4 kb and 6.0 kb; KSU5, *Eco*RI, 9.8 kb, 7.6 kb, 6.1 kb, 3.8 kb and 3.5 kb; UMC130, *Eco*RI*,* 13.5 kb and 7.0 kb; UMC130, *Hind*III, 4.8 kb and 3.2 kb; UMC130, *Bam*HI*,* 3.2 kb; UMC152, *Hind*III*,* 12.4, 7.1 kb and 5.6 kb; UMC64, *Hind*III*,* 3.3 kb; phi06005, *Eco*RI*,* 12.8 kb; UMC163, *Hind*III*,* 7.0 kb, 4.8 kb and 2.6 kb; UMC44, *Hind*III, 9.8 kb, 8.7 kb, 7.2 kb, 5.5 kb and 4.0 kb; BNL10.13, *Hind*III*,* 10.8 kb; npi306, *Hind*III, 7.0 kb; pmt1, *Hind*III, 2.3 kb; pmt2, *Hind*III, 8.0 kb, 4.2 kb, 2.8 kb and 2.1 kb; pmt5, *Hind*III, 12.3 kb, 8.1 kb, 3.6 kb, 3.2 kb and 2.5 kb; tda48, *Hind*III, 8.2 kb; tda53; *Hind*III, 3.8 kb and 2.2 kb; tda168. *Eco*RI, 3.6 kb; tda16. *Hind*III, 4.3 kb; and tda17, *Hind*III, 7.0 kb.

The invention also provides:
- a method of providing hybrid maize seed that contains one or more restriction fragments described above, comprising the steps of :
   (a) crossing a *Tripsacum* female parent with a *Tripsacum* pollen donor plant to produce hybrid seed;
   (b) growing a hybrid plant from said seed to maturity;
   (c) screening said hybrid plant to determine whether the plant is a cross between *Tripsacum* and perennial teosinte using the method described above;
   (d) crossing said *(Tripsacum* X perennial teosinte) or (perennial teosinte X *Tripsacum)* hybrid plant with maize to produce seed; and
   (e) harvesting the seed produced in (d);
- a method of producing a hybrid maize plant that contains one or more restriction fragments set out above, which method comprises:
   (a) producing hybrid maize seed using a method as set out above; and
   (b) growing the hybrid maize seed to give a hybrid maize plant; and
- a method for producing a derivative, variant, mutant, modification, cellular component, molecular component, pollen or tissue culture from a hybrid maize plant, which method comprises:
   (a) producing hybrid maize seed using a method as set out above;
   (b) growing the hybrid maize seed to give a hybrid maize plant; and
   (c) obtaining a derivative, variant, mutant, modification, cellular component, molecular component, pollen or tissue culture from the hybrid maize plant.

The intergeneric hybrids obtainable using the methods referred to above are fully fertile and cross-fertile with maize. The hybrid plants are characterized by their utility as a genetic bridge to transfer novel genetic materials into maize and their unexpected chromosome number of 2n=20 instead of the expected 2n=28 or 2n=46 if a full, unmodified complement of perennial teosinte haploid chromosomes (n=10) and diploid *Tripsacum* (n=18) or tetraploid *Tripsacum* (n=36) chromosomes were transmitted to the resulting hybrid progeny.

An intergeneric hybrid plant *(Tripsacum* X perennial teosinte) or (perennial teosinte X *Tripsacum)* is crossed with maize by controlled pollination. In the cross, the pollen of *(Tripsacum* X perennial teosinte) or (perennial teosinte X *Tripsacum)* is transferred to maize silks, or maize pollen is transferred to the silks of *(Tripsacum* X perennial teosinte) or (perennial teosinte X *Tripsacum).*

Hybrid seed, hybrid plants produced by the seed and/or tissue culture, variants, mutants, modifications, and cellular and molecular components of the hybrid plants that contain novel genetic materials derived from ( *Tripsacum* X perennial teosinte) or (perennial teosinte X *Tripsacum)* may be obtained.

In the method of the invention for producing hybrid maize seed, the trigeneric hybrid plant obtained from crossing *(Tripsacum* X perennial teosinte) or (perennial teosinte X *Tripsacum )* with maize by controlled pollination, may be backcrossed to maize or ( *Tripsacum* X perennial teosinte) or (perennial teosinte X *Tripsacum).* In the backcross, the pollen of the trigeneric hybrid plant is transferred to the silks of one of the original parents *(Tripsacum* X perennial teosinte) or (perennial teosinte X *Tripsacum)* or maize.

This invention thus allows the preparation of hybrid seed, hybrid plants produced by the seed and/or tissue culture, variants, mutants, modifications, and cellular and molecular components of the hybrid plants that contain novel genetic materials derived from *(Tripsacum* X perennial teosinte) or (perennial teosinte X *Tripsacum).*

Plants and plant tissues produced by the method of crossing maize with a *Tripsacum*-diploperennis hybrid contain novel genetic materials and exhibit beneficial agronomic traits. For example, these plants may contain novel genes for such traits as pest and pathogen resistance, drought tolerance, cold tolerance, water-logging tolerance, improved grain quality, improved forage quality, totipotency, perennialism, tolerance to acidic soils, tolerance to high-aluminum soils, enhanced adaptability in a carbon dioxide enriched environment and can be employed in recurrent selection breeding programs to select for hybrid maize that exhibit such traits.

For the purposes of this application, the following terms are defined to provide a clear and consistent description of the invention.

Allele. One of the different forms of a gene that can exist at a single locus.

Autoradiography. A process in which radioactive materials are incorporated into cellular components, then placed next to a film or photographic emulsion to produce patterns on the film that correspond to the location of the radioactive compounds within the cell.

Electrophoresis. A technique for separating the components of a mixture of molecules (proteins, DNAs, or RNAs) in an electric field within a gel matrix.

Genetic markers. Alleles used as experimental probes to keep track of an individual, a tissue, a cell, a nucleus, a chromosome, or a gene.

Gene. The fundamental physical and functional unit of heredity that carries information from one generation to the next. The plant gene is "a DNA sequence of which a segment is regularly or conditionally transcribed at some time in either or both generations of the plant. The DNA is understood to include not only the exons and introns of the structural gene but the cis 5' and 3' regions in which a sequence change can affect gene expression" (Neuffer, Coe and Wessler 1997).

Genotype. The allelic composition of a cell - either of the entire cell or, more commonly, for a certain gene or a set of genes of an individual.

Hybrid plant. An individual plant produced by crossing two parents of different genotypes or germplasm backgrounds.

Locus. The place on a chromosome where a gene is located.

Molecular genetics. The study of the molecular processes underlying gene structure and function.

Mutagen. An agent that is capable of increasing the mutation rate.

Mutation. (1) The process that produces nucleotide sequences, genes, genetic elements, or chromosomes differing from the wild-type. (2) The nucleotide sequences, genes, genetic elements, or chromosomes that result from such a process.

Plant breeding. The application of genetic analysis to development of plant lines better suited for human purposes.

Probe. Defined nucleic acid segment that can be used to identify specific molecules bearing the complementary DNA or RNA sequence, usually through autoradiography.

Restriction enzyme. An endonuclease that will recognize specific target nucleotide sequences in DNA and cut the DNA at these points; a variety of these enzymes are known and they are extensively used in genetic engineering.

RFLP. Refers to restriction fragment length polymorphism that is a specific DNA sequence revealed as a band of particular molecular weight size on a Southern blot probed with a radiolabelled RFLP probe and is considered to be an allele of a gene.

Southern blot. Transfer of electrophoretically separated fragments of DNA from the gel to an absorbent surface such as paper or a membrane which is then immersed in a solution containing a labeled probe that will bind to homologous DNA sequences.

Totipotency. The ability of a cell to proceed through all the stages of development and thus produce a normal adult.

Wild type - refers to a reference and it can mean an organism, set of genes, gene or nucleotide sequence. For purposes herein the wild type refers to the parents of hybrid progeny.

### DESCRIPTION OF THE DRAWING

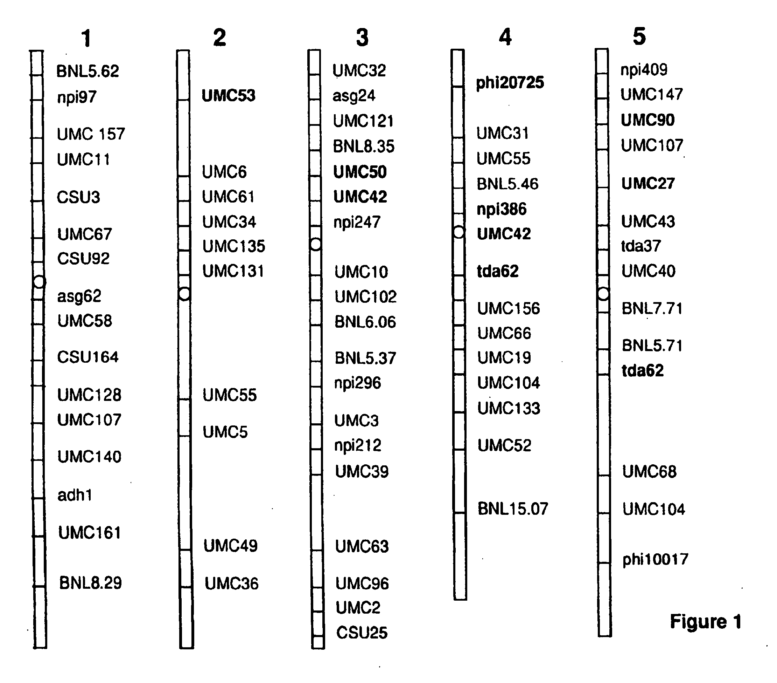

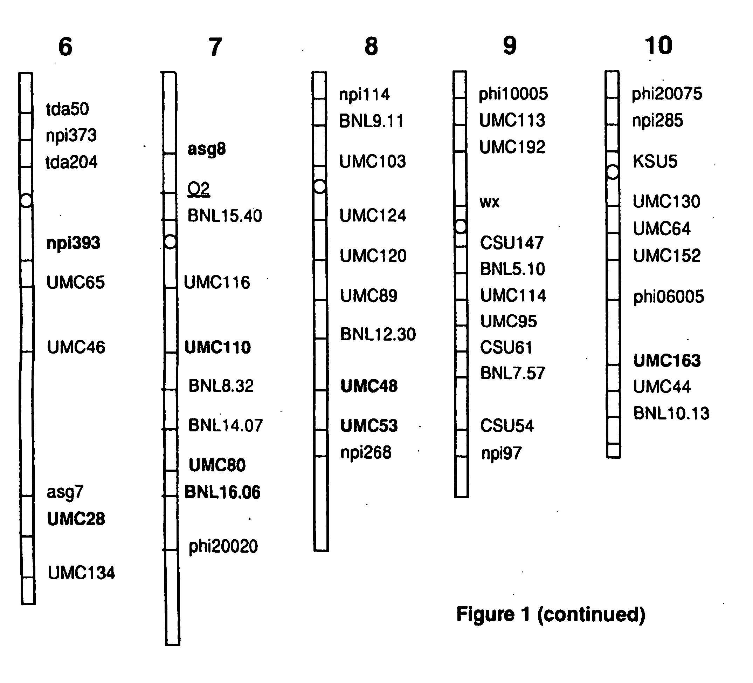

Figure 1 is a schematic drawing of the 10 linkage groups of maize. The open circles represent approximate positions of the centromeres. The labelled lines indicate positions where the probes used to DNA fingerprint hybrid plants derived from crossing *Tripsacum dactyloides* and *Zea diploperennis,* as well as maize plants crossed with the *Tripsacum*-diploperennis bridging species are located on maize chromosomes. Molecular markers that reveal loci where stable, heritable de novo alleles are underscored, and loci where stable, heritable new alleles from *Tripsacum* that are not found in maize or the wild Zeas are italicized.

### DETAILED DESCRIPTION OF THE INVENTION

The principles and techniques used to identify de novo genetic material and novel *Tripsacum* alleles are central to molecular genetics and are commonly used to fingerprint crop varieties (Kresovich et al. 1993). First DNA is extracted and isolated from plant samples; then the DNA is cut into fragments using restriction enzymes that cut at precise nucleotide sequences; the fragments are then separated by size, i.e. molecular weight, on an agarose gel by electrophoresis; the DNA is then denatured, i.e. separated into single strands, and transferred to a nitrocellulose filter which binds single-stranded but not double-stranded DNA, a method referred to as Southern blotting. The restriction fragments are immobilized on the filter in a pattern that mirrors their positions in the agarose gel. The membrane is then incubated in a solution containing multiple copies of a radiolabeled probe for a particular DNA sequence that has been mapped to a certain chromosomal locus or loci in the maize genome; the probe hybridizes to homologous DNA sequences, and the distinctive banding pattern formed by a particular restriction enzyme/probe combination in any individual plant can be visualized on an autoradiograph. The banding patterns, which resemble a bar code, precisely identify the genotype of individual plants. The patterns formed by specific restriction enzyme/probe combinations are referred to as restriction fragment length polymorphisms (RFLPs), and they provide sufficient information to distinguish between plants whose genetic composition may differ slightly. These genetic fingerprinting techniques permit the unambiguous identification of genotypes (Melchinger et al. 1991; Messmer et al. 1993). Fingerprinting profiles are routinely used for genetic identity analysis to classify closely related materials, estimate genetic distances between such materials, determine paternity, and complement conventional pedigree records in commercial hybrid production (Smith and Smith 1992).

Since maize is a diploid organism, the progeny of maize hybrids inherit one allele for a trait from one parent and another allele for that trait from the other parent. In the DNA fingerprint of a single gene that is not duplicated elsewhere in the genome, if the progeny inherits the same allele for a trait from both parents, it is homozygous for that trait and a single band will be present on the DNA profile using a molecular probe that maps to the specific region of the particular chromosome to which the trait being investigated has been mapped. If the progeny inherits different alleles from each parent plant, it will be heterozygous at that locus and two bands will be detected on the autoradiograph, one band from one parent and a different band from the other parent. At more complex loci involving gene duplication, multiple bands can be seen. In general, the offspring of two parents can be identified by comparing the banding pattern profile because the progeny exhibit a combination of bands from both parents. Sometimes, however, the progeny of known parentage exhibit a band or bands that are not found in either parent. Such de novo bands arise from mutable or recombinant events that give rise to changes in the nucleotide sequences such that the banding pattern is different from both of its parents (Griffiths et al. 1993). Such mutant or novel rearrangements in the genetic material are revealed by comparative analysis of the banding patterns of the parent plants and hybrid progeny. Bands present in the progeny not found in either parent indicate regions of the genome where novel genetic material has arisen, i.e. mutations have occurred.

As stated above, mutations are rare, and in most cases are deleterious. Broadly speaking among all organisms, mutation rates vary and they range from 1 in 1,000 to 1 in 1,000,000 gametes per generation depending on the gene involved (Curtis and Barnes 1989). For example, each human with approximately 100,000 genes is expected to carry 2 mutant alleles. The *Tripsacum-*perennial teosinte hybrids are unprecedented in that their DNA fingerprints reveal they have an extremely high mutation rate with de novo alleles at 133 out of 173 loci. Furthermore, de novo alleles are stably inherited in succeeding generations of *Tripsacum-*diploperennis progeny and of maize X *Tripsacum*-diploperennis progeny. In addition to the rarity and usual deleterious effect of mutations, a basic biological tenet is that mutations occur at random or by chance (Lewin 1997). In a study of spontaneous mutation rates to new length alleles at tandemly repeated loci in human DNA (Jeffreys et al. 1988) mutations arose sporadically and there was no clustering of mutations within a family. Siblings never shared a common mutant allele. Therefore, it is unexpected that the same mutations would recur among siblings or among hybrids of different parentage. Thus it is remarkable and unexpected that the same de novo alleles are repeatedly found in hybrid progeny derived from crossing different *Tripsacum* and perennial teosinte parent plants, and that those same de novo alleles are stably inherited in crosses between *Tripsacum*-perennial teosinte hybrids and maize. These de novo alleles provide a rich new source of variant genetic material for selection in corn improvement.

In molecular assays performed by Linkage Genetics, Salt Lake City, Utah, and Biogenetics, Inc., Brookings, South Dakota, DNA was isolated from different F₁, F₂ and F₃ hybrids between *Tripsacum dactyloides* and *Zea diploperennis,* the parents of these hybrids, W64A and B73 maize inbred lines, as well as F₁, F₂, F₃ and F₄ hybrids between maize and *Tripsacum-*diploperennis. The protocol for DNA isolation, restriction enzyme digestion, Southern blotting, probe hybridization, and analysis of autoradiographs has been described by Helentjaris et al. (1986). Internal standards of known molecular weights and a ladder were included in the gels to facilitate accuracy of describing the banding patterns in terms of molecular weights of alleles. Five different *Tripsacum*-diploperennis hybrids have been fingerprinted: (1) Sun Dance, Zea *diploperennis* 3-7 X *Tripsacum dactyloides* (2n=72); (2) Tripsacorn, *Tripsacum dactyloides* (2n=72) X Zea *diploperennis* 3-3; (3) Sun Star, Zea *diploperennis* 2-4 X *Tripsacum dactyloides* (2n=36); (4) Sun Devil, *Tripsacum dactyloides* (2n=72) X Zea *diploperennis;* (5) 20A self, *Zea diploperennis* 2-4 X *Tripsacum dactyloides* (2n=72). The maize inbred lines W64A and B73 were crossed with some of the above *Tripsacum*-diploperennis hybrids.

Total genomic DNA from individuals in some of the above listed lines was digested with from one to four different restriction enzymes, *Eco*RI, *Eco*RV, *Hin*dIII, and *Bam*I, transferred to Southern blots, and probed with 173 publicly available DNA markers which include a majority of maize nuclear DNA probes mapped to the ten linkage groups of maize (Gardiner et al. 1993), six maize mitochondrial probes, and some *Tripsacum* (tda) probes for which the loci have not yet been mapped to the maize genome. The molecular markers on the genetic linkage map of maize were mapped by recombinational analyses based on proof of the identity of a clone. Thus each locus represents a gene based on clone identification (Neuffer, Coe and Wessler 1997). The 173 molecular markers that were employed in DNA fingerprinting of parent species, *Tripsacum-*diploperennis hybrids, and maize X *Tripsacum*-diploperennis are listed in Table 1. Figure 1 depicts the orders and approximate locations of the mapped probes on the ten maize chromosomes (cf. Neuffer, Coe and Wessler 1997). A large number of the probes reveal bands that are not present in either parent of a particular progeny. These de novo bands signal loci where mutations occurred in the process of intergeneric hybridization. Their approximate mapped loci on the ten chromosomes of Zea are shown in Figure 1, and they are indicated in Table 1 by underscoring. There are also loci where *Tripsacum* alleles are present in *Tripsacum-*diploperennis hybrids that are not present in maize or the wild *Zeas.* Thus these are novel alleles that can now be conferred to maize via the *Tripsacum*-perennial teosinte genetic bridge. These are italicized in Table 1 and highlighted in bold face type in Figure 1.

Table 2 lists the approximate size, i.e. molecular weight, of each de novo band per restriction enzyme/probe combination that has arisen as a result of the mutagenic effect of the wide cross hybridization. Table 3 lists approximate size of the novel *Tripsacum* alleles not found in maize or the wild *Zeas* that can now be conferred to maize via *Tripsacum*-diploperennis hybrid lines according to restriction enzyme/probe combination.

Tables 4 identifies the mutant nucleotide sequences, and specifies their inheritance in *Tripsacum-*diploperennis hybrids and eight exemplary (maize X *Tripsacum*-diploperennis) lines. Table 5 identifies the nucleotide sequences that are alleles from the *Tripsacum* parents employed in producing the *Tripsacum*-diploperennis hybrids and specifies their inheritance in the *Tripsacum-*diploperennis hybrids and eight exemplary (maize X *Tripsacum-*diploperennis) lines. In order to determine which *Tripsacum* alleles are present in *Tripsacum*-diploperennis hybrids that are not present in other *Zeas*, 5 to 13 individuals from populations of two modern maize inbred lines, B73 and W64A, four indigenous Latin American maize races, Nal Tel (Yuc7), Chapalote (Sin), Pollo (Col 35 ICA), and Pira (PI44512), and the six wild *Zeas, Z. mexicana* (PI566683 and PI566688), *Z. parviglumis* (PI384061 and PI331785), *Z. luxurians* (PI306615), *Z. huehuetenangensis* (Ames21880), *Z.* diploperennis and *Z. perennis*(Ames 21875), were DNA fingerprinted with the probes in Table 1 and Figure 1. The nucleotide sequences are identified by probe/restriction enzyme/probe and molecular weight.

The novel genetic materials, including de novo alleles and new alleles from *Tripsacum,* that are not found in maize or the wild Zeas, have been shown to be stably inherited in three generations of *Tripsacum-*diploperennis hybrids, and four generations of *Tripsacum*-diploperennis hybrids that were crossed with maize. The new *Tripsacum* alleles and mutated nucleotide sequences, their heritability in succeeding generations of *Tripsacum*-diploperennis hybrids, and their transmissibility to maize is unprecedented and unexpected based on prior art. These novel DNA sequences have utility for genetic analysis of Zea, and selection of new variant alleles that may enhance traits such as insect and disease resistance, drought stress tolerance, cold tolerance, perennialism, increased grain yield, totipotency, apomixis, better root systems, tolerance of water-logged soils, tolerance of high-aluminum and acidic soils, improved grain quality, and improved forage quality. New traits derived from these mutations or novel *Tripsacum* genes can be successfully employed in recurrent selection breeding programs for maize improvement.

The method of the invention is performed by crossing *Tripsacum dactyloides* and *Zea diploperennis.* The crosses are performed using standard plant breeding techniques for controlled pollinations known in the art. Some *Tripsacum*-diploperennis hybrid plants which are perennials that reproduce asexually as well as by seed have been described in the following plant patents: PP No. 9,640 issued September 3, 1996; PP No. 7,977 issued September 15, 1992, and PP No. 6,906 issued July 4, 1989. U.S. Patent No. 5,330,547 issued July 19, 1994, and U.S. Patent No. 5,750,828 issued May 12, 1998, describe a method for employing *Tripsacum-*diploperennis hybrids to confer corn rootworm resistance to maize.

The present invention provides a method of obtaining novel genetic materials, including de novo mutant nucleotide sequences and new alleles from *Tripsacum* that are not found in maize or the wild *Zeas,* by producing hybrid plant seeds comprising the steps of (a) pollinating a *Tripsacum* species (e.g. *Tripsacum dactyloides)* female parent with pollen from a perennial teosinte species (e.g. Zea *diploperennis*) male parent, or of pollinating a perennial teosinte species (e.g. Zea *diploperennis)* female parent with pollen from a *Tripsacum* species (e.g. *Tripsacum dactyloides),* to produce seed; then (b) harvesting the seed produced.

This method produces a hybrid seed from which a hybrid plant containing novel genetic materials can be grown, and from hybrid plants containing novel genetic material tissue cultures can be made. Additionally, pollen produced by the hybrid plant containing novel genetic material can be collected.

The term "plant" as used in this application refers to the whole plant as well as its component parts, e.g., flowers, roots, fruits, stems, rhizomes, pollen.

The present invention further provides a method of producing hybrid maize seed containing novel genetic materials comprising the steps of (a) crossing a *Tripsacum* pollen recipient plant with a perennial teosinte pollen donor to plant produce *(Tripsacum* X perennial teosinte), or a perennial teosinte pollen recipient plant with a *Tripsacum* pollen donor plant (perennial teosinte X *Tripsacum),* to produce hybrid seed; then (b) growing a (*Tripsacum* X perennial teosinte) or (perennial teosinte X *Tripsacum)* hybrid plant from said seed to maturity; then (c) crossing said *(Tripsacum* X perennial teosinte) or (perennial teosinte X *Tripsacum*) hybrid plant with either a maize pollen recipient or maize pollen donor plant to produce seed and (d) harvesting the seed produced.

This method results in the production of hybrid maize seed and hybrid maize plants containing novel genetic materials, from which tissue cultures can be made. Plant breeding techniques and tissue culture techniques as described herein are known, and may be carried out in the manner known to those skilled in the art. See, for example, U.S. Patent No. 4,737,596 to Seifert et al. entitled "Hybrid Maize Plant and Seed"; U.S. Patent No. 5,059,745 to Foley entitled "Hybrid Maize Line LH195"; U.S. Patent No. 4,545,146 to Davis entitled "Route to Hybrid Soybean Production"; U.S. Patent No. 4,627,192 to Fick entitled "Sunflower Products and Methods for their Production", and U.S. Patent Nos. 4,837,152 and 4,684,612 entitled "Process for Regenerating Soybeans"; U.S. Patent Nos. 5,330,547 and 5,750,828 to Eubanks entitled "Methods and Materials for Conferring Tripsacum Genes in Maize."

In *Tripsacum* inflorescences, the staminate (i.e. male) flowers and pistillate (i.e. female) flowers are produced on a single spike with the male flowers subtended by the female. When *Tripsacum* sends out the inflorescence, the staminate flowers are broken off leaving only the female flowers on the spike which are then covered with a pollinating bag, i.e. standard ear shoot bag for maize, to protect them from contamination by unwanted pollen. Perennial teosinte male and female flowers occur on separate parts of the plant. The staminate flowers are borne in the tassel which emerges at the apex of the culm; whereas, the pistillate flowers occur in single-rowed spikes borne on lateral branches of the culm. When perennial teosinte produces its tassels, they are covered with a pollinating bag. When they start shedding pollen, the bag is removed and pollen taken to pollinate the *Tripsacum* plants. At that time, the bags covering the *Tripsacum* pistillate flowers are removed and the perennial teosinte pollen shaken out of the bag onto the silks. The *Tripsacum* inflorescence is covered again with a pollinating bag immediately after pollination and the bag is stapled so that it remains on the spike until the seed has matured. Upon maturity, approximately 45 days later, the seed is harvested. Once mature seed from the cross has been obtained, it is planted, and the plants from seed that germinates are grown in a growth chamber, greenhouse or the field. Controlled crosses are best made in a greenhouse or growth chamber where plants are kept isolated to prevent cross contamination and there is no problem with bags being damaged by weather conditions.

This method may alternatively be used to cross the plants with perennial teosinte as the female parent. In this embodiment, all the tassels, i.e. male flowers, are removed from the perennial teosinte plant as soon as they emerge and the ears, i.e. female flowers, are covered with pollinating bags. Rather than removing *Tripsacum* male flowers, the spikes are left in tact and covered with a pollinating bag to collect *Tripsacum* pollen. The pollen is applied to the diploperennis ears which are then immediately covered with a pollinating bag that is well fastened with staples to ensure it remains sealed until the seed has matured, approximately 45 days after pollination when the seed is harvested.

Next, when (*Tripsacum* X perennial teosinte) or (perennial teosinte X *Tripsacum*) starts to flower, the same steps described above are used to cross the hybrid with maize. To cross onto maize, as soon as the maize plants begin to produce ears, before the silks emerge, the ears are covered with an ear shoot bag. Pollen collected from *(Tripsacum* X perennial teosinte) or (perennial teosinte X *Tripsacum)* is applied to silks of the maize ears. The ears are then covered again with an ear shoot bag and a large pollinating bag which is wrapped around the culm and secured with a staple. The ears remain covered until they reach maturity, several weeks later when the ears are harvested.

To pollinate the (*Tripsacum* X perennial teosinte) or (perennial teosinte X *Tripsacum*) hybrid with maize pollen, the tassel of the maize plant is covered with a large pollinating bag, a day or two before collection. Pistillate flowers of Tripsacum-diploperennis hybrid plants frequently have staminate tips above the female flowers as described for *Tripsacum.* Whenever *Tripsacum-*perennial teosinte plants are to be pollinated by another plant, all the staminate tips are removed as soon as the ears emerge to prevent possibility of self pollination. The pistillate flowers of the hybrid are covered with an ear shoot bag as soon as they begin to appear on the plant but before the silks emerge. Pollen collected from maize is applied to silks of the hybrid female spikes which are then immediately covered with an ear shoot bag that is stapled closed. The ears remain covered until they reach maturity, approximately 45 days later, and then the seed is harvested.

Plants obtained from all crosses described above are male and female fertile, are cross-fertile with each other, are cross-fertile with maize, and carry novel genetic material, i.e. new alleles from *Tripsacum* that are not present in maize and the wild *Zeas* and de novo alleles derived from mutations that arose in the process of intergeneric hybridization, as identified in DNA fingerprints employing 173 different molecular probes distributed throughout the ten linkage groups of maize.

The examples and embodiments described herein illustrate the invention.

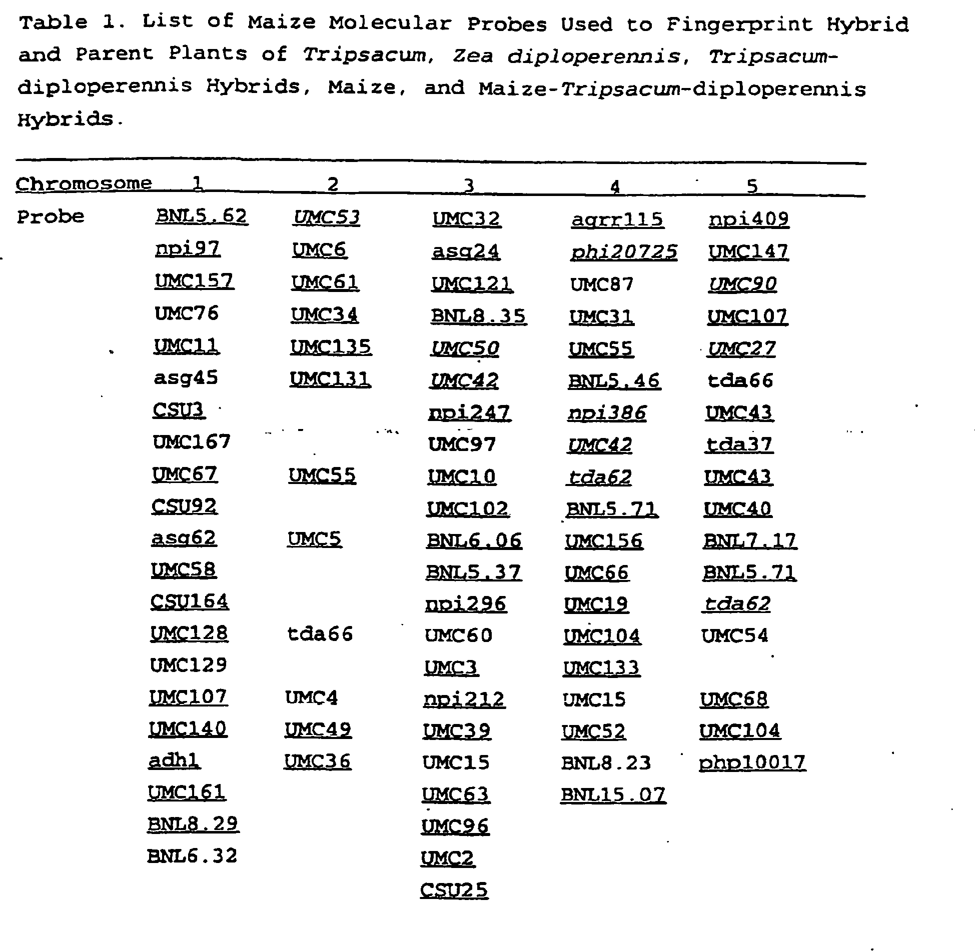

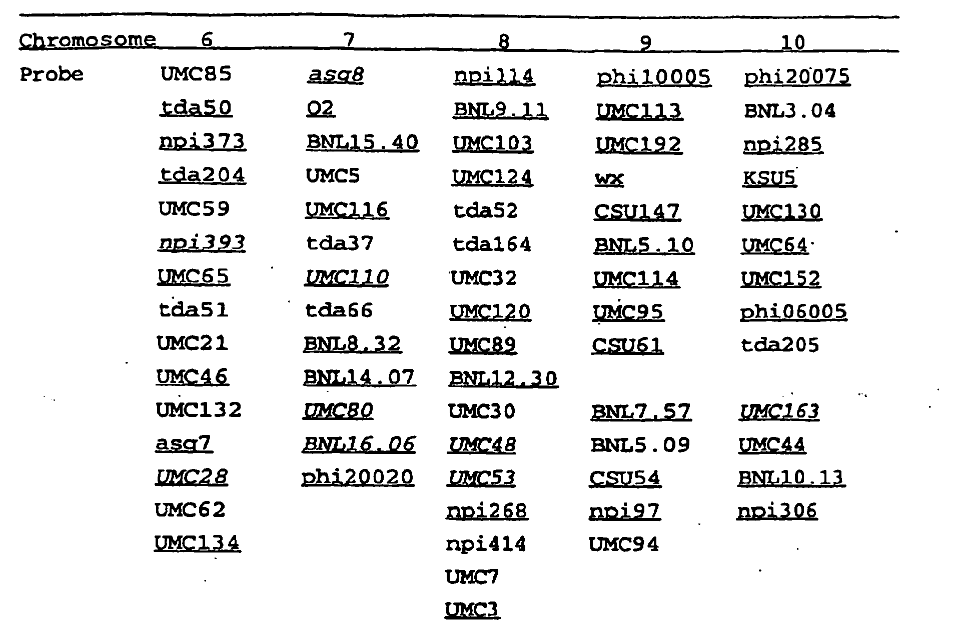

| Other Probes | | |
|---|---|---|
| | | |

| Mitochondrial | | Locus unknown |
|---|---|---|
| Probe | pmt1 | tda16 |
| | pmt2 | tda17 |
| | pmt3 | tda48 |
| | pmt4 | tda53 |
| | *pmt5* | tda80 |
| | pmt6 | *tda168* |
| | | tda250 |

**Table 2. Approximate Size of De Novo Restriction Length Fragment Polymorphisms Per Enzyme/Probe Combination.**

| | Restriction Enzyme | | | |
|---|---|---|---|---|
| Probe | *Eco*RI | *Hind*III | *Bam*HI | *Eco*RV |
| **Chromosome 1** | | | | |
| BNL5.62 | 10.3Kb | - | - | - |
| npi97 | - | 3.9Kb | - | - |
| UMC157 | 6.5Kb | 5.5Kb | 14.0Kb | - |
| | 3.3Kb | | 8.6Kb | |
| | | | 4.5Kb | |
| UMC11 | - | - | 7.0Kb | - |
| CSU3 | - | - | 10.0Kb | - |
| | | | 7.6Kb | |
| | | | 3.5Kb | |
| UMC67 | 19.2Kb | 23.1Kb | 13.4Kb | - |
| | | | 11.0Kb | |
| | | | 1.6Kb | |
| CSU92 | - | - | 13.3Kb | - |
| | | | 7.5Kb | |
| asg62 | - | - | 12.7Kb | - |
| | | | 9.7Kb | |
| | | | 6.6Kb | |
| UMC58 | 15.3Kb | 3.3Kb | - | - |
| CSU164 | 9.0Kb | - | - | - |
| | 7.0Kb | | | |
| UMC128 | - | 6.0Kb | - | - |
| UMC107 | 6.3Kb | 19.2Kb | - | - |
| | 5.3Kb | | | |
| UMC140 | 23.0Kb | 6.5Kb | - | - |
| | 9.0Kb | | | |
| | 5.0Kb | | | |
| adhl | - | 9.4Kb | 9.4Kb | - |
| UMC161 | - | 3.3Kb | 15.3Kb | - |
| | | | 8.0Kb | |
| BNL8.29 | - | 9.3Kb | - | - |
| | | 8.3Kb | | |

| **Chromosome 2** | | | | |
|---|---|---|---|---|
| UMC53 | 9.4Kb | - | - | 3.8Kb |
| | | | | 3.0Kb |
| UMC6 | 3.8Kb | 9.4Kb | 15.3Kb | - |
| | | | 13.2Kb | |
| | | | 12.7Kb | |
| | | | 10.0Kb | |
| | | | 7.0Kb | |
| UMC61 | - | 3.4Kb | - | - |
| | | 2.8Kb | | |
| UMC³⁴ | 7.5Kb | 8.8Kb | 9.4Kb | - |
| | 5.4Kb | 6.5Kb | | |
| | | 5.8Kb | | |
| UMC135 | - | 11.6Kb | - | - |
| | | 10.8Kb | | |
| UMC131 | 10.6Kb | - | - | - |
| | 5.8Kb | | | |
| | 4.3Kb | | | |
| UMC55 | 3.9Kb | 4.3Kb | - | - |
| UMC5 | 5.4Kb | 6.5Kb | - | - |
| UMC49 | - | - | 8.2Kb | - |
| | | | 6.0Kb | |
| | | | 4.2Kb | |
| | | | 3.2Kb | |
| UMC36 | - | - | 4.2Kb | - |

| **Chromosome 3** | | | | |
|---|---|---|---|---|
| UMC32 | 5.3Kb | 6.7Kb | - | - |
| asg24 | - | 7.2Kb | - | - |
| | | 6.4Kb | | |
| UMC121 | 3.7Kb | - | - | - |
| | 3.2Kb | | | |
| BNL8.35 | - | 9.9Kb | - | - |
| | | 8.7Kb | | |
| UMC50 | - | - | 6.8Kb | - |
| | | | 3.8Kb | |
| UMC42 | - | 10.4Kb | - | - |
| | | 8.9Kb | | |
| | | 3.7Kb | | |
| | | 3.0Kb | | |
| npi247 | 8.0Kb | 3.0Kb | - | - |
| UMC10 | 6.5Kb | 5.9Kb | - | - |
| | 5.5Kb | 3.0Kb | | |
| UMC102 | 2.7Kb | - | - | - |
| BNL6.06 | 6.8Kb | - | - | - |
| BNL5.37 | - | 10.3Kb | - | - |
| | | 5.8Kb | | |
| | | 3.5Kb | | |
| npi296 | 7.9Kb | - | - | - |
| UMC3 | 2.5Kb | - | - | - |
| | 2.0Kb. | | | |
| npi212 | - | 4.3Kb | 5.4Kb | - |
| UMC39 | 12.2Kb | - | - | - |
| | 9.2Kb | | | |
| | 7.8Kb | | | |
| | 7.1Kb | | | |
| UMC63 | - | 9.5Kb | - | - |
| | | 4.3Kb | | |
| UMC96 | - | 11.8Kb | 7.5Kb | - |
| | | 6.4Kb | | |
| | | 5.5Kb | | |
| UMC2 | 11.8Kb | - | - | - |
| | 10.4Kb | | | |
| | 8.0Kb | | | |
| CSU25 | - | 4.6Kb | - | - |
| | | 4.3Kb | | |

| **Chromosome 4** | | | | |
|---|---|---|---|---|
| agrr115 | 8.0Kb | 19.2Kb | 5.4Kb | - |
| | 5.4Kb | | 3.5Kb | - |
| phi20725 | 10.3Kb | 1.5Kb | - | - |
| | 7.2Kb | | | |
| UMC31 | 5.8Kb | - | 6.5Kb | - |
| UMC55 | 3.9Kb | 4.3Kb | - | - |
| BNL5.46 | - | 13.7Kb | - | - |
| | | 10.5Kb | | |
| | | 9.7Kb | | |
| | | 5.1Kb | | |
| npi386 | - | 9.3Kb | - | - |
| | | 8.2Kb | | |
| UMC42 | - | 19.2Kb | - | - |
| | | 10.3Kb | | |
| | | 8.9Kb | | |
| | | 3.7Kb | | |
| | | 3.0Kb | | |
| tda62 | - | - | 5.5Kb | - |
| BNL5.71 | - | - | - | 11.3Kb |
| | | | | 6.8Kb |
| | | | | 5.7Kb |
| UMC156 | | 3.OKb | - | - |
| UMC66 | 10.5Kb | - | 3.7Kb | - |
| UMC19 | - | - | 12.3Kb | - |
| UMC104 | - | 12.4Kb | - | |
| | | 11. 6Kb | | |
| | | 7.5Kb | | |
| UMC133 | - | 10.6Kb | - | - |
| | | 9.9Kb | | |
| | | 9.2Kb | | |
| | | 7.7Kb | | |
| UMC52 | - | - | 8.7Kb | - |
| | | | 6.9Kb | |
| | | | 3.8Kb | |
| | | | 3.0Kb | |
| | | | 2.0Kb | |
| BNL15.07 | - | 2.9Kb | - | - |
| | | 2.7Kb | | |

| **Chromosome 5** | | | | |
|---|---|---|---|---|
| npi409 | 9.4Kb | 10.4Kb | 19.2Kb | - |
| | | 9.0Kb | | |
| | | 3.9Kb | | |
| | | 3.0Kb | | |
| UMC147 | - | 16.3Kb | - | - |
| | | 3.8Kb | | |
| | | 2.4Kb | | |
| UMC90 | - | 2.8Kb | 9.0Kb | - |
| | | 2.5Kb | | |
| | | 1.6 | | |
| UMC107 | 6.3Kb | - | - | - |
| UMC27 | - | 4.5Kb | 6.5Kb | - |
| tda37 | - | - | 8.2Kb | - |
| | | | 6.5Kb | |
| UMC43 | - | - | 9.7Kb | - |
| | | | 7:3Kb | |
| | | | 9.7Kb | |
| UMC40 | - | - | 7.2Kb | - |
| | | | 4.3Kb | |
| | | | 4.0Kb | |
| BNL7.71 | - | 10.6Kb | - | - |
| BNL5.71 | - | - | - | 11.3Kb |
| | | | | 6.8Kb |
| | | | | 5.7Xb |
| tda62 | - | - | 5.5Kb | - |
| UMC68 | - | 6.0Kb | - | - |
| UMC104 | - | 12.4Kb | 9.4Kb | - |
| | | 11.6Kb | | |
| | | 7.5Kb | | |
| phi10017 | - | - | - | 15.1Kb |
| | | | | 9.5Kb |

| **Chromosome 6** | | | | |
|---|---|---|---|---|
| tda50 | - | - | 8.5Kb | - |
| npi373 | - | 6.5Kb | - | - |
| | | 5.6Kb | | |
| | | 3.0Kb | | |
| tda204 | - | - | 4.0Kb | - |
| npi393 | 12.1Kb | - | - | - |
| | 8.5Kb | | | |
| | 5.6Kb | | | |
| UMC65 | - | 2.9Kb | - | - |
| UMC21 | 5.7Kb | - | - | - |
| UMC46 | 6.5Kb | - | - | - |
| | 5.6Kb | - | - | - |
| asg7 | - | 6.3Kb | - | - |
| UMC28 | - | 15.8Kb | 7.6Kb | - |
| | | 11.9Kb | 6.6Kb | |
| UMC134 | 15.3Kb | 7.5Kb | 4.7Kb | - |
| | | | | |

| **Chromosome 7** | | | | |
|---|---|---|---|---|
| asg8 | - | 10.8Kb | - | - |
| | | 8.4Kb | | |
| O₂ | 9.4Kb | - | - | - |
| BNL15.40 | - | 5.8Kb | - | - |
| UMC116 | 9.5Kb | 15.3Kb | - | - |
| UMC110 | - | - | 10.6Kb | - |
| | | | 4.9Kb | |
| BNL8.32 | - | 8.9Kb | - | |
| | | 7.4Kb | | |
| | | 7.1Kb | | |
| BNL14.07 | 6.4Kb | - | - | - |
| UMC80 | - | 2.4Kb | - | - |
| BNL16.06 | 6.8Kb | - | - | - |
| phi20020 | - | 7.8Kb | - | - |
| | | 6.6KB | | |

| **Chromosome 8** | | | | |
|---|---|---|---|---|
| npi114 | - | 10.0Kb | - | - |
| | | 8.8Kb | | |
| | | 6.3KB | | |
| BLNL9.11 | - | 3.4Kb | - | - |
| UMC103 | - | 6.9Kb | - | - |
| | | 5.7Kb | | |
| UMC124 | - | 8.0Kb | 21.0Kb | - |
| | | 7.0Kb | 19.0Kb | |
| | | | 6.6Kb | |
| | | | 2.6Kb | |
| | | | 1.6Kb | |
| UMC120 | - | 8.0Kb | 23.1Kb | - |
| | | 3.2Kb | | |
| | | 2.3Kb | | |
| | | 1.4Kb | | |
| UMC89 | 7.3Kb | 7.3Kb | 9.5Kb | - |
| | | | 6.0Kb | |
| | | | 5.2Kb | |
| | | | 4.5Kb | |
| BNL12.30 | 3.5Kb | - | - | - |
| UMC48 | - | 4.7Kb | - | - |
| | | 3.5Kb | | |
| | | 2.2Kb | | |
| UMC53 | 3.8Kb | - | - | - |
| | 3.0Kb | | | |
| npi268 | - | - | 6.4Kb | - |
| UMC3 | 2.5Kb | - | - | - |
| | 2.0Kb | | | |

| **Chromosome 9** | | | | |
|---|---|---|---|---|
| phi10005 | 15.0Kb | - | - | - |
| UMC113 | 5.9Kb | - | 12.8Kb | - |
| | 5.4Kb | | 11.8Kb | |
| | | | 10.5Kb | |
| UMC192 | - | 11.4Kb | - | - |
| | | 6.4Kb | | |
| wx (waxy) | - | 21.0Kb | - | - |
| CSU147 | - | 5.9Kb | - | - |
| BNL5.10 | - | 6.1Kb | - | - |
| | | 4.4Kb | | |
| UMC114 | - | - | 15.0Kb | - |
| | | | 12.6Kb | |
| | | | 11.5Kb | |
| | | | 10.0Kb | |
| | | | 8.8Kb | |
| | | | 7.5Kb | |
| | | | 6.5Kb | |
| UMC95 | 13.3Kb | 7.7Kb | 15.0Kb | - |
| | 5.6Kb | 4.8Kb | 9.0Kb | |
| | | 4.1Kb | | |
| CSU61 | 8.1Kb | - | - | - |
| | 4.8Kb | | | |
| bn17.57 | 1.0Kb | - | 11.6Kb | - |
| | | | 5.9Kb | |
| | | | 5.5Kb | |
| | | | 1.3Kb | |
| CSU54 | 14.7Kb | - | - | - |
| | 12.6Kb | | | |

| **Chromosome 10** | | | | |
|---|---|---|---|---|
| phi20075 | 7.1Kb | - | - | - |
| npi285 | 15.3Kb | - | - | - |
| | 12.4Kb | | | |
| | 9.4Kb | | | |
| | 6.OKb | | | |
| KSUS | 9.8Kb | - | - | - |
| | 7.6Kb | | | |
| | 6.1Kb | | | |
| | 3.8Kb | | | |
| | 3.5Kb | | | |
| UMC130 | 13.5Kb | 4.8Kb | 3.2Kb | - |
| | 7.0Kb | 3.2Kb | | |
| UMC152 | - | 12.4Kb | - | - |
| | | 7.1Kb | | |
| | | 5.6Kb | | |
| UMC64 | - | 3.3Kb | - | - |
| phi06OO5 | 12.8Kb | - | - | - |
| UMC163 | - | 12.0Kb | - | - |
| | | 7.0Kb | | |
| | | 4.8Kb | | |
| UMC44 | - | 9.8Kb | - | - |
| | | 8.7Kb | | |
| | | 7.2Kb | | |
| | | 5.5Kb | | |
| | | 4.0Kb | | |
| BNL10.13 | - | 10.8Kb | - | - |
| npi306 | - | 7.0Kb | - | - |
| | | | | |

| **Mitochondria** | | | | |
|---|---|---|---|---|
| pmt1 | - | 2.3Kb | - | - |
| pmt2 | - | 8.0Kb | - | - |
| | | 4.2Kb | | |
| | | 2.8Rb | | |
| | | 2.1Kb | | |
| pmt5 | - | 12.3Kb | - | - |
| | | 8.1Kb | | |
| | | 3.2Kb | | |
| | | 2.5Kb | | |

| **Map Location Unknown** | | | | |
|---|---|---|---|---|
| tda16 | - | 4.3Kb | - | - |
| tda17 | - | 7.0Kb | - | - |
| tda37 | - | - | 8.2Kb | - |
| | | | 6.5Kb | |
| tda48 | - | 8. 2Kb | - | - |
| tda53 | - | 3.8Kb | - | - |
| | | 2.2Kb | | |

| Table 3. Approximate Size of New Tripsacum Restriction Length Fragment Polymorphisms Per Enzyme/Probe Combination Not Present in Maize or the Wild Zeas. | | | | |
|---|---|---|---|---|
| | Restriction Enzyme | | | |
| Probe | *Eco*RI | *Hind*III | *Bam*HI | EcoRV |
| **Chromosome 2** | | | | |
| UMC53 | - | - | - | 8.4Kb |
| | | | | |

| **Chromosome 3** | | | | |
|---|---|---|---|---|
| UMC50 | - | - | 7.8Kb | - |
| | | | 5.8Kb | |
| UMC42 | - | 7.6Kb | - | - |
| | | | | |

| **Chromosome 4** | | | | |
|---|---|---|---|---|
| phi20725 | 9.7Kb | - | - | - |
| npi386 | - | 12.6Kb | - | - |
| UMC42 | - | 7.6Kb | - | - |
| tda62 | - | - | 4.8Kb | - |
| | | | | |

| **Chromosome 5** | | | | |
|---|---|---|---|---|
| UMC90 | - | 7.8Kb | - | - |
| UMC27 | - | 8.3Kb | - | - |
| | | | | |

| **Chromosome 6** | | | | |
|---|---|---|---|---|
| tda50 | - | - | 6.8Kb | - |
| npi393 | 7.0Kb | - | - | - |
| UMC28 | - | - | 10.0Kb | - |
| | | | | |

| **Chromosome 7** | | | | |
|---|---|---|---|---|
| asg8 | - | 8.7Kb | - | - |
| UMC110 | - | - | 3.9Kb | - |
| UMC80 | - | 10.6Kb | - | - |
| BNL16.06 | - | 1.9Kb | - | - |
| | | | | |

| **Chromosome 8** | | | | |
|---|---|---|---|---|
| UMC48 | - | 6.2Kb | - | - |
| UMC53 | - | - | - | 8.4Kb |
| | | | | |

| **Chromosome 10** | | | | |
|---|---|---|---|---|
| UMC163 | - | 2.6Kb | - | - |
| | | | | |

| **Mitochondria** | | | | |
|---|---|---|---|---|
| pmt5 | - | 3.6Kb | - | - |
| | | | | |

| **Map Location Unknown** | | - | - | - |
|---|---|---|---|---|
| tda168 | 3.6Kb | | | |

**Table 4. De Novo Alleles in Tripsacum -diploperennis Hybrids and (Maize X Tripsacum -diploperennis)**

| ***Probe*/*Enzyme*** | | ***Tripsacum* -diploperennis Hybrids** | | | | | **Maize X *Tripsacum* -diploperennis** | | | | | | | |
|---|---|---|---|---|---|---|---|---|---|---|---|---|---|---|
| **Chromosome 1** | | **Sun Dance** | **20A** | **Tripsacorn** | **Sun Star** | | **64SS** | **64TC** | **2019** | **3024** | **3028** | **3125** | **4126** | **TC64** |
| BNL5.62-ERI | | 10.3kb | | | | | | | 10.3kb | 10.3kb | 10.3kb | | | |
| npi97-H | | 3.9kb | | 3.9kb | | | | | 3.9kb | 3.9kb | 3.9kb | 3.9kb | | |
| UMC157-ERI | | 6.5kb | | 6.5kb | | | | 6.5kb | | | | | | |
| UMC157-ERI | | 3.3kb | | 3.3kb | | | | 3.3kb | | | | | | |
| UMC157-H | | 5.5kb | | 5.5kb | | | | 5.5kb | | | | | | |
| UMC157-B | | 14.0kb | 14.0kb | 14.0kb | 14.0kb | | 14.0kb | 14.0kb | | | 14.0kb | | | |
| UMC157-B | | | 8.6kb | | 8.6kb | | | | | | 8.6kb? | | | |
| UMC157-B | | | | 4.5kb | 4.5kb | | | | 4.5kb | | | | | 4.5kb |
| UMC11-B | | 7.0kb | 7.0kb | 7.0kb | 7.0kb | | | | 7.0kb | 7.0kb | | 7.0kb | | 7.0kb |
| CSU3-B | | | | 10.0kb | 10.0kb | | | | 10.0kb | 10.0kb | | | 10.0kb | 10.0kb |
| CSU3-B | | | | 7.6kb | 7.6kb | | | | 7.6kb | 7.6kb | | | 7.6kb | 7.6kb |
| CSU3-B | | 3.5kb | | 3.5kb | | | | | | | | 3.5kb | | 3.5kb |
| UMC67-ERI | | | | | 19.2kb | | | | | | | | | |
| UMC67-H | | | | | 23.1 kb | | | | | | | | | |
| UMC67-B | | 13.4kb | | | | | | | | | | | | |
| UMC67-B | | 11.0kb | 11.0kb | 11.0kb | 11.0kb | | | | 11.0kb | 11.0kb | 11.0kb | 11.0kb | 11.0kb | 11.0kb |
| UMC67-B | | | | | 1.6kb | | | | | | | | | |
| CSU92-B | | 13.3kb | | 13.3kb | 13.3kb | | | | 13.3kb | 13.3kb | | | | 13.3kb |
| CSU92-B | | 7.5kb | 7.5kb | | | | | | | | | | | |
| asg62-B | | 12.7kb | 12.7kb | 12.7kb | | | | | | | | | | |
| asg62-B | | | 9.7kb | 9.7kb | 9.7kb | | | | 9.7kb | 9.7kb | 9.7kb | | 9.7kb | |
| asg62-B | | 6.6kb | | 6.6kb | | | | | | | | | | |
| UMC58-ERI | | 15.3kb | | 15.3kb | 15.3kb | | 15.3kb | 15.3kb | | | | | | |
| UMC58-H | | 3.3kb | | 3.3kb | 3.3kb | | 3.3kb | 3.3kb | | | | | | |
| CSU164-ERI | | 9.0kb | | 9.0kb | 9.0kb | | | | 9.0kb | 9.0kb | | | 9.0kb | |
| CSU164-ERI | | | 7.0kb | | | | | | | | | | | |
| UMC128-H | | | | 6.0kb | 6.0kb | | | | 6.0kb | 6.0kb | | | 6.0kb | 6.0kb |
| UMC107-ERI | | | | 6.3kb | 6.3kb | | 6.3kb | | | | | | | |
| UMC107-ERI | | 5.3kb | | 5.3kb | | | | 5.3kb | | | | | | |
| UMC107-H | | 19.2kb | | 19.2kb | | | | | | | | | | |
| UMC140-ERI | | 23.0kb | | | | | | | | | | | | |
| UMC140-ERI | | 9.0kb | | 9.0kb | | | | | | | | | | |
| UMC140-ERI | | 5.0kb | | 5.0kb | 5.0kb | | | | 5.0kb | 5.0kb | 5.0kb | | | 5.0kb |
| UMC140-H | | 6.5kb | | 6.5kb | | | | | | | | | | |
| adh1-H | | 9.4kb | | 9.4kb | 9.4kb | | | | | | | | | |
| adh1-B | | 9.4kb | | 9.4kb | 9.4kb | | 9.4kb | 9.4kb | | | | | | |
| UMC161-H | | 3.3kb | | 3.3kb | 3.3kb | | 3.3kb | 3.3kb | | | | | | |
| UMC161-B | | 15.3kb | | | | | | | | | | | | |
| UMC161-B | | | | 8.0kb | 8.0kb | | | | | | | | | |
| BNL8.29-H | | | | 9.3kb | | | | | | | | | 9.3kb | |
| BNL8.29-H | | 8.3kb | 8.3kb | 8.3kb | 8.3kb | | | | | | | | | 8.3kb |
| | | | | | | | | | | | | | | |

| **Chromosome 2** | | | | | | | | | | | | | | |
|---|---|---|---|---|---|---|---|---|---|---|---|---|---|---|
| UMC53-ERI | | 9.4kb | | 9.4kb | 9.4kb | | 9.4kb | | | | | | | |
| UMC53-ERV | | | 3.8kb | 3.8kb | 3.8kb | | | | 3.8kb | 3.8kb | 3.8kb | | | 3.8kb |
| UMC53-ERV | | 3.0kb | | | | | | | | | | | | |
| UMC6-ERI | | | | 3.8kb | | | | | | | | | | |
| UMC6-H | | | | 9.4kb | 9.4kb | | 9.4kb | | | | | | | |
| UMC6-B | | | | 15.3kb | | | | | | | | | | |
| UMC6-B | | 13.2kb | 13.2kb | | | | | | | | | | | |
| UMC6-B | | | | 12.7kb | | | | | | | | | | |
| UMC6-B | | | 10.0kb | | | | | | | | | | | |
| UMC6-B | | 7.0kb | 7.0kb | | | | | | | | | | | |
| UMC61-H | | | | 3.4kb | 3.4kb | | | | | | | | | |
| UMC61-H | | 2.8kb | | 2.8kb | 2.8kb | | | | 2.8kb | 2.8kb | 2.8kb | 2.8kb | 2.8kb | 2.8kb |
| UMC34-ERI | | 7.5kb | | | | | | | | | | | | |
| UMC34-ERI | | 5.4kb | | 5.4kb | 5.4kb | | 5.4kb | | | | | | | |
| UMC34-H | | 8.8kb | | 8.8kb | 8.8kb | | 8.8kb | | | | | | | |
| UMC34-H | | 6.5kb | | 6.5kb | | | | | | | | | | |
| UMC34-H | | 5.8kb | 5.8kb | | | | | | 5.8kb | 5.8kb | | 5.8kb | 5.8kb | 5.8kb |
| UMC34-B | | | | 9.4kb | 9.4kb | | 9.4kb | | | | | | | |
| UMC135-H | | | 11.6kb | | | | | | | | | | | |
| UMC135-H | | | | 10.8kb | | | | | 10.8kb | 10.8kb | | 10.8kb | | |
| UMC131-ERI | | | | 10.6kb | | | | | | | | | | |
| UMC131-ERI | | 5.8kb | | | | | | | | | | | | |
| UMC131-ERI | | 4.3kb | 4.3kb | 4.3kb | 4.3kb | | 4.3kb | 4.3kb | 4.3kb | 4.3kb | 4.3kb | | | |
| UMC55-ERI | | 3.9kb | 3.9kb | 3.9kb | | | | | 3.9kb | 3.9kb | | 3.9kb | 3.9kb | 3.9kb |
| UMC55-H | | 4.3kb | | 4.3kb | | | | | | | | | | |
| UMC5-ERI | | 5.4kb | | 5.4kb | 5.4kb | | 5.4kb | | | | | | | |
| UMC5-H | | | | 6.5kb | 6.5kb | | 6.5kb | | | | | | | |
| UMC49-B | | | 8.2kb | | 8.2kb | | | | | | | | | |
| UMC49-B | | | 6.0kb | 6.0kb | 6.0kb | | | | | | | | | |
| UMC49-B | | | | 4.2kb | 4.2kb | | | | | 4.2kb | | 4.2kb | 4.2kb | |
| UMC49-B | | 3.2kb | | | | | | | | | | | | |
| UMC36-B | | 4.2kb | | | | | | | | | | | | |
| | | | | | | | | | | | | | | |

| **Chromosome 3** | | | | | | | | | | | | | | |
|---|---|---|---|---|---|---|---|---|---|---|---|---|---|---|
| UMC32-ERI | | 5.3kb | | 5.3kb | 5.3kb | | 5.3kb | 5.3kb | 6.7kb | 6.7kb | | 6.7kb | 6.7kb | 6.7kb |
| UMC32-H | | 6.7kb | | 6.7kb | 6.7kb | | | | | | | | | |
| asg24-H | | 7.2kb | 7.2kb | 7.2kb | 7.2kb | | | | | | 7.2kb | 7.2kb | 7.2kb | 7.2kb |
| asg24-H | | 6.4kb | | 6.4kb | 6.4kb | | | | | | | 6.4kb | | 6.4kb |
| UMC121-ERI | | 3.7kb | 3.7kb | 3.7kb | 3.7kb | | | | | 3.7kb | 3.7kb | | | 3.7kb |
| UMC121-ERI | | 3.2kb | 3.2kb | | 3.2kb | | | | | | | | | |
| BNL8.35-H | | | | 9.9kb | | | | | | | | | | |
| BNL8.35-H | | | 8.7kb | | | | | | | | | | | |
| UMC50-B | | 6.8kb | | | | | | | | | | | | |
| UMC50-B | | | | 3.8kb | | | | | | | 3.8kb | 3.8kb | 3.8kb | |
| UMC42-H | | 10.4kb | 10.4kb | 10.4kb | 10.4kb | | | | 10.4kb | 10.4kb | 10.4kb | 10.4kb | 10.4kb | 10.4kb |
| UMC42-H | | | 8.9kb | | 8.9kb | | | | | | 8.9kb | | | |
| UMC42-H | | 3.7kb | | 3.7kb | | | | | 3.7kb | 3.7kb | 3.7kb | 3.7kb | 3.7kb | 3.7kb |
| UMC42-H | | 3.0kb | | | | | | | | | | | | |
| npi247-ERI | | 8.0kb | | 8.0kb | 8.0kb | | 8.0kb | | | | | | | |
| npi247-H | | | | 3.0kb | 3.0kb | | 3.0kb | | | | | | | |
| UMC10-ERI | | | | 6.5kb | 6.5kb | | 6.5kb | | | | | | | |
| UMC10-ERI | | | | 5.5kb | 5.5kb | | 5.5kb | | | | | | | |
| UMC10-H | | 5.9kb | | | 5.9kb | | | | | | | | | |
| UMC10-H | | 3.0kb | | | 3.0kb | | | | | | | | | |
| UMC102-ERI | | | 2.7kb | | 2.7kb | | | | | | 2.7kb | | | |
| BNL6.06-ERI | | 6.8kb | 6.8kb | 6.8kb | 6.8kb | | | | | 6.8kb | 6.8kb | | | 6.8kb |
| BNL5.37-H | | | | 10.3kb | 10.3kb | | | | 10.3kb | 10.3kb | 10.3kb | 10.3kb | 10.3kb | 10.3kb |
| BNL5.37-H | | 5.8kb | 5.8kb | 5.8kb | 5.8kb | | | | | | 5.8kb | | | 5.8kb |
| BNL5.37-H | | 3.5kb | 3.5kb | 3.5kb | 3.5kb | | | | 3.5kb | 3.5kb | 3.5kb | 3.5kb | 3.5kb | 3.5kb |
| npi296-ERI | | 7.9kb | | 7.9kb | 7.9kb | | 7.9kb | 7.9kb | | | | | | |
| UMC3-ERI | | 2.5kb | | 2.5kb | 2.5kb | | | | 2.5kb | 2.5kb | | 2.5kb | 2.5kb | 2.5kb |
| UMC3-ERI | | 2.0kb | 2.0kb | | 2.0kb | | | | | | | | | |
| npi212-H | | | | 4.3kb | 4.3kb | | | | | | | | | |
| npi212-B | | | | 5.4kb | 5.4kb | | | | | | | | | |
| UMC39-ERI | | 12.2kb | 12.2kb | | | | | | | | 12.2kb | | | |
| UMC39-ERI | | | 9.2kb | | 9.2kb | | | | | | 9.2kb | | | |
| UMC39-ERI | | 7.8kb | 7.8kb | | 7.8kb | | | | | | | | | |
| UMC39-ERI | | 7.1 kb | | 7.1 kb | 7.1 kb | | | | | 7.1 kb | | 7.1 kb | 7.1 kb | 7.1kb |
| UMC63-H | | | | | 9.5kb | | | | 9.5kb | | 9.5kb | 9.5kb | 9.5kb | 9.5kb |
| UMC63-H | | 4.3kb | 4.3kb | | 4.3kb | | | | | | 4.3kb | | | |
| UMC96-H | | | | 11.8kb | 11.8kb | | | | 11.8kb | 11.8kb | | | | |
| UMC96-H | | | | | 6.4kb | | | | | | 6.4kb | | | |
| UMC96-H | | 5.5kb | | 5.5kb | 5.5kb | | | | | | | | | |
| UMC96-B | | 7.5kb | | | | | | | | | | | | |
| UMC2-ERI | | 11.8kb | | 11.8kb | | | | | | | | 11.8kb | 11.8kb | |
| UMC2-ERI | | | | | 10.4kb | | | | | | | | | |
| UMC2-ERI | | | 8.0kb | | 8.0kb | | | | | | 8.0kb | | | |
| CSU25-H | | 4.6kb | | | | | | | | | | 4.6kb | 4.6kb | 4.6kb |
| CSU25-H | | 4.2kb | 4.2kb | | 4.2kb | | | | | | 4.2kb | | | |
| | | | | | | | | | | | | | | |

| **Chromosome 4** | | | | | | | | | | | | | | |
|---|---|---|---|---|---|---|---|---|---|---|---|---|---|---|
| agrr115-ERI | | | | 8.0kb | 8.0kb | | 8.0kb | 8.0kb | | | | | | |
| agrr115-ERI | | 5.4kb | | 5.4kb | | | 5.4kb | 5.4kb | | | | | | |
| agrr115-H | | | | 19.2kb | 19.2kb | | 19.2kb | | | | | | | |
| agrr115-B | | | | 5.4kb | 5.4kb | | 5.4kb | 5.4kb | | | | | | |
| agrr115-B | | | | 3.5kb | 3.5kb | | 3.5kb | 3.5kb | | | | | | |
| phi20725-ERI | | | | 10.3kb | 10.3kb | | | | | | | | | 10.3kb |
| phi20725-ERI | | | 7.2kb | | 7.2kb | | | | | 7.2kb | | | | |
| phi20725-H | | 1.5kb | | | | | | | | | | | | |
| UMC31-ERI | | | | 5.8kb | 5.8kb | | 5.8kb | | | | | | | |
| UMC55-ERI | | 3.9kb | 3.9kb | 3.9kb | | | | | 3.9kb | 3.9kb | | 3.9kb | 3.9kb | 3.9kb |
| UMC55-H | | 4.3kb | | 4.3kb | | | | | | | | | | |
| BNL5.46-B | | 13.7kb | | | | | | | | | | | | |
| BNL5.46-B | | | 10.5kb | | 10.5kb | | | | | | | | | |
| BNL5.46-B | | 9.7kb | | 9.7kb | 9.7kb | | | | 9.7kb | 9.7kb | 9.7kb | 9.7kb | 9.7kb | 9.7kb |
| BNL5.46-B | | | | 5.1kb | | | | | | | 5.1 kb | | | |
| npi386-H | | 9.3kb | 9.3kb | | | | | | | | | | | |
| npi386-H | | 8.2kb | | 8.2kb | | | | | | | | 8.2kb | | |
| UMC42-H | | 19.2kb | | 19.2kb | 19.2kb | | 19.2kb | 19.2kb | | | | | | |
| UMC42-H | | 10.3kb | 10.3kb | 10.3kb | 10.3kb | | | | 10.3kb | 10.3kb | 10.3kb | 10.3kb | 10.3kb | 10.3kb |
| UMC42-H | | | 8.9kb | | 8.9kb | | | | | | 8.9kb | | | |
| UMC42-H | | 3.7kb | | 3.7kb | | | | | 3.7kb | 3.7kb | 3.7kb | 3.7kb | 3.7kb | 3.7kb |
| UMC42-H | | 3.0kb | | | | | | | | | | | | |
| tda62-B | | 5.5kb | 5.5kb | 5.5kb | | | | | | | | 5.5kb | | |
| BNL5.71-ERV | | 11.3kb | 11.3kb | 11.3kb | 11.3kb | | | | | | | | | 11.3kb |
| BNL5.71-ERV | | | 6.8kb | | 6.8kb | | | | | | 6.8kb | | | |
| BNL5.71-ERV | | 5.7kb | | 5.7kb | | | | | 5.7kb | 5.7kb | | 5.7kb | 5.7kb | 5.7kb |
| UMC156-H | | 3.0kb | | 3.0kb | 3.0kb | | 3.0kb | | | | | | | |
| UMC66-ERI | | | | 10.5kb | 10.5kb | | | | | | | | | |
| UMC66-B | | 3.7kb | 3.7kb | 3.7kb | 3.7kb | | | | 3.7kb | 3.7kb | 3.7kb | 3.7kb | 3.7kb | 3.7kb |
| UMC19-B | | 12.3kb | 12.3kb | 12.3kb | 12.3kb | | | | 12.3kb | 12.3kb | 12.3kb | | 12.3kb | 12.3kb |
| UMC104-H | | | | | 12.4kb | | | | | | | | | |
| UMC104-H | | 11.6kb | | 11.6kb | | | | | | | | | | |
| UMC104-H | | | 7.5kb | | | | | | | | | | | |
| UMC133-H | | 10.6kb | 10.6kb | 10.6kb | | | | | | | | | | |
| UMC133-H | | | | | 9.9kb | | | | | | | | | |
| UMC133-H | | | | 9.2kb | | | | | | 9.2kb | 9.2kb | 9.2kb | 9.2kb | |
| UMC133-H | | | | | 7.7kb | | | | | | | | | |
| UMC52-B | | | | 8.7kb | 8.7kb | | | | 8.7kb | 8.7kb | | 8.7kb | 8.7kb | 8.7kb |
| UMC52-B | | 6.9kb | | | | | | | | | | | | |
| UMC52-B | | 3.8kb | | 3.8kb | | | | 3.8kb | | | | | | |
| UMC52-B | | 3.0kb | | | | | | | | | | | | |
| UMC52-B | | | | 2.0kb | 2.0kb | | 2.0kb | | | | | | | |
| BNL15.07-H | | 2.9kb | | | | | | | | | | | | |
| BNL15.07-H | | | | | 2.7kb | | | | | | | | | |
| | | | | | | | | | | | | | | |

| **Chromosome 5** | | **Sun Dance** | **20 A** | **Tripsacorn** | **Sun Star** | | **64SS** | **64TC** | **2019** | **3024** | **3028** | **3125** | **4126** | **TC64** |
|---|---|---|---|---|---|---|---|---|---|---|---|---|---|---|
| npi409-ERI | | 9.4kb | | 9.4kb | | | | | | | | | | |
| npi409-H | | | | 10.4kb | | | | | | | | | | |
| npi409-H | | | | 9.0kb | 9.0kb | | 9.0kb | | | | | | | |
| npi409-H | | 3.9kb | 3.9kb | 3.9kb | 3.9kb | | | 3.9kb | 3.9kb | 3.9kb | 3.9kb | 3.9kb | 3.9kb | 3.9kb |
| npi409-H | | 3.0kb | | 3.0kb | 3.0kb | | 3.0kb | 3.0kb | | | | | | |
| npl409-B | | | | 19.2kb | 19.2kb | | 19.2kb | | | | | | | |
| UMC147-H | | | 16.3kb | | 16.3kb | | | | 16.3kb | | 16.3kb | | | |
| UMC147-H | | 3.8kb | | | | | | | | | | | | |
| UMC147-H | | 2.4kb | | 2.4kb | | | | | 2.4kb | 2.4kb | | 2.4kb | | |
| UMC90-H | | 2.8kb | | | | | | | | | | | | |
| UMC90-H | | 2.5kb | | | 2.5kb | | | | | | | | | |
| UMC90-H | | 1.6kb | | | | | | | | | | | | |
| UMC90-B | | | | 9.0kb | 9.0kb | | 9.0kb | | | | | | | |
| UMC107-ERI | | 6.3kb | | | 6.3kb | | | | | | 6.3kb | | | |
| UMC27-H | | 4.5kb | | | | | | | | | | | | |
| UMC27-B | | 6.5kb | | | | | | | | | | | | |
| tda37-B | | 8.2kb | | | | | | | | | | | | |
| tda37-B | | 6.5kb | | | | | | | | | | | | |
| UMC43-B | | | 9.7kb | | 9.7kb | | | | | | 9.7kb | | | |
| UMC43-B | | 7.3kb | 7.3kb | 7.3kb | 7.3kb | | | | 7.3kb | 7.3kb | | 7.3kb | 7.3kb | 7.3kb |
| UMC43-B | | 5.7kb | | | | | | | | | | | | |
| UMC40-B | | 7.2kb | | | | | | | | | | | | |
| UMC40-B | | | | 4.3kb | 4.3kb | | | | 4.3kb | | | | 4.3kb | 4.3kb |
| UMC40-B | | 4.0kb | 4.0kb | 4.0kb | 4.0kb | | | | | 4.0kb | 4.0kb | 4.0kb | | |
| BNL7.71-H | | | | 10.6kb | | | | | | | | | 10.6kb | 10.6kb |
| BNL5.71-B | | 11.3kb | 11.3kb | 11.3kb | 11.3kb | | | | | | | | | 11.3kb |
| BNL5.71-B | | | 6.8kb | | 6.8kb | | | | | | 6.8kb | | | |
| BNL5.71-B | | 5.7kb | | 5.7kb | | | | | 5.7kb | 5.7kb | | 5.7kb | 5.7kb | 5.7kb |
| tda62-B | | 5.5kb | 5.5kb | 5.5kb | | | | | | | | 5.5kb | | |
| UMC68-H | | 6.0kb | 6.0kb | 6.0kb | 6.0kb | | | | 6.0kb | 6.0kb | 6.0kb | | 6.0kb | 6.0kb |
| UMC104-H | | | | | 12.4kb | | | | | | | | | |
| UMC104-H | | 11.6kb | | 11.6kb | | | | | | | | | | |
| UMC104-H | | | 7.5kb | | | | | | | | | | | |
| UMC104-B | | 9.4kb | | 9.4kb | 9.4kb | | 9.4kb | 9.4kb | | | | | | |
| phi 10017-B | | | | 15.1 kb | | | | | | | | | | |
| phi10017-B | | 9.5kb | 9.5kb | 9.5kb | 9.5kb | | | | 9.5kb | 9.5kb | 9.5kb | | 9.5kb | |
| | | | | | | | | | | | | | | |

| **Chromosome 6** | | | | | | | | | | | | | | |
|---|---|---|---|---|---|---|---|---|---|---|---|---|---|---|
| tda50-B | | 8.5kb | 8.5kb | 8.5kb | 8.5kb | | | | | 8.5kb | 8.5kb | 8.5kb | 8.5kb | 8.5kb |
| npi373-H | | 6.5kb | | 6.5kb | 6.5kb | | | | 6.5kb | 6.5kb | | 6.5kb | 6.5kb | 6.5kb |
| npi373-H | | | 5.6kb | | 5.6kb | | | | 5.6kb | 5.6kb | 5.6kb | | | |
| npi373-H | | 3.0kb | | | | | | | | | | | | |
| tda204-B | | | | 4.0kb | | | | | 4.0kb | 4.0kb | | | | 4.0kb |
| NPI393-ERI | | 12.1kb | | 12.1 kb | | | | | 12.1kb | 12.1kb | | 12.1kb | 12.1kb | |
| NPI393-ERI | | 8.5kb | 8.5kb | 8.5kb | 8.5kb | | | | | | 8.5kb | | | 8.5kb |
| NPI393-ERI | | 5.6kb | 5.6kb | | | | | | | | | | | |
| UMC65-H | | 2.9kb | | | | | | | | | | | | |
| UMC46-ERI | | 6.5kb | | 6.5kb | 6.5kb | | 6.5kb | 6.5kb | | | | | | |
| UMC46-ERI | | 5.6kb | 5.6kb | 5.6kb | 5.6kb | | | | 5.6kb | 5.6kb | 5.6kb | 5.6kb | 5.6kb | 5.6kb |
| asg7-H | | 6.3kb | | | | | | | | | | | | |
| UMC28-H | | | | 15.8kb | 15.8kb | | | | | | | | | |
| UMC28-H | | | | 11.9kb | 11.9kb | | | | | | | | | |
| UMC28-B | | | | 7.6kb | 7.6kb | | | | 7.6kb | 7.6kb | 7.6kb | 7.6kb | 7.6kb | 7.6kb |
| UMC28-B | | | | 6.6kb | 6.6kb | | | | 6.6kb | | | | | |
| UMC134-ERI | | 15.3kb | | 15.3kb | 15.3kb | | 15.3kb | 15.3kb | | | | | | |
| UMC134-H | | 7.5kb | | 7.5kb | 7.5kb | | 7.5kb | 7.5kb | | | | | | |
| UMC134-H | | 4.7kb | 4.7kb | | 4.7kb | | | | | | 4.7kb | | | |
| | | | | | | | | | | | | | | |
| | | | | | | | | | | | | | | |

| **Chromosome 7** | | **Sun Dance** | **20A** | **Tripsacorn** | **Sun Star** | | **64SS** | **64TC** | **2019** | **3024** | **3028** | **3125** | **4126** | **TC64** |
|---|---|---|---|---|---|---|---|---|---|---|---|---|---|---|
| asg8-H | | 10.8kb | | 10.8kb | | | | | | | | | | |
| asg8-H | | | 8.4kb | | | | | | | | | | | |
| O2-ERI | | 9.4kb | | 9.4kb | 9.4kb | | | | | | | | | |
| BNL15.40-H | | 5.8kb | 5.8kb | 5.8kb | | | | | | | | | 5.8kb | |
| UMC116-ERI | | 9.5kb | 9.5kb | 9.5kb | 9.5kb | | | | | | | | 9.5kb | |
| UMC116-H | | 15.3kb | | | | | | | | | | | | |
| UMC110-B | | 10.6kb | 10.6kb | 10.6kb | 10.6kb | | | | 10.6kb | 10.6kb | 10.6kb | 10.6kb | 10.6kb | 10.6kb |
| UMC110-B | | 4.9kb | 4.9kb | 4.9kb | 4.9kb | | | | 4.9kb | 4.9kb | | 4.9kb | 4.9kb | |
| BNL8.32-H | | | | 8.9kb | | | | | 8.9kb | | 8.9kb | | | 8.9kb |
| BNL8.32-H | | 7.4kb | | | | | | | | | | | | |
| BNL8.32-H | | | 7.1 kb | 7.1 kb | 7.1 kb | | | | | | | | | |
| BNL14.07-ERI | | | 6.4kb | | 6.4kb | | | | | | | | | |
| UMC80-H | | 7.9kb | 7.9kb | 7.9kb | 7.9kb | | | | | | | | 7.9kb | |
| UMC80-H | | 2.4kb | 2.4kb | | 2.4kb | | | | | 2.4kb | 2.4kb | | | |
| BNL16.06-ERI | | 6.8kb | 6.8kb | 6.8kb | 6.8kb | | | | | 6.8kb | 6.8kb | | | 6.8kb |
| phi20020-H | | 7.8kb | 7.8kb | 7.8kb | 7.8kb | | | | 7.8kb | 7.8kb | 7.8kb | 7.8kb | 7.8kb | 7.8kb |
| phi20020-H | | 6.6kb | | | | | | | | | | | | |

| | | | | | | | | | | | | | | |
|---|---|---|---|---|---|---|---|---|---|---|---|---|---|---|
| **Chromosome 8** | | **Sun Dance** | **20A** | **Tripsacorn** | **Sun Star** | | **64SS** | **64TC** | **2019** | **3024** | **3028** | **3125** | **4126** | **TC64** |
| npi114-H | | | | 10.0kb | 10.0kb | | | | | | | 10.0kb | 10.0kb | 10.0kb |
| npi114-H | | 8.8kb | | 8.8kb | 8.8kb | | | | | 8.8kb | 8.8kb | | | 8.8kb |
| npi114-H | | | | 6.3kb | | | | | | | | | | 6.3kb |
| BNL9.11-H | | | 3.4kb | | | | | | | | | | | |
| UMC103-H | | | 6.9kb | 6.9kb | 6.9kb | | | | 6.9kb | 6.9kb | 6.9kb | 6.9kb | 6.9kb | 6.9kb |
| UMC103-H | | 5.7kb | | | | | | | | | | | | |
| UMC124-H | | | | 8.0kb | 8.0kb | | 8.0kb | 8.0kb | | | | | | |
| UMC124-H | | 7.0kb | | 7.0kb | | | | 7.0kb | | | | | | |
| UMC124-B | | 21.0kb | | 21.0kb | 21.0kb | | 21.0kb | 21.0kb | | | | | | |
| UMC124-B | | 19.0kb | | 19.0kb | 19.0kb | | 19.0kb | 19.0kb | | | | | | |
| UMC124-B | | | | 6.6kb | 6.6kb | | | | | | | | | |
| UMC124-B | | | 2.6kb | 2.6kb | 2.6kb | | | | 2.6kb | 2.6kb | | | | 2.6kb |
| UMC124-B | | 1.6kb | 1.6kb | 1.6kb | 1.6kb | | | | 1.6kb | 1.6kb | 1.6kb | 1.6kb | 1.6kb | 1.6kb |
| UMC120-H | | | | 8.0kb | 8.0kb | | 8.0kb | 8.0kb | | | | | | |
| UMC120-H | | 3.2kb | | 3.2kb | 3.2kb | | | | 3.2kb | | 3.2kb | 3.2kb | 3.2kb | 3.2kb |
| UMC120-H | | 2.3kb | | | | | | | | | | | | |
| UMC120-H | | | 1.4kb | 1.4kb | 1.4kb | | | | | | | | | 1.4kb |
| UMC120-B | | | | 23.1 | | | | | | | | | | |
| UMC89-ERI | | | | | 7.3kb | | | | | | | | | |
| UMC89-H | | | | | 7.3kb | | | | | | | | | |
| UMC89-B | | | | 9.5kb | 9.5kb | | 9.5kb | | 9.5kb | 9.5kb | | | | 9.5kb |
| UMC89-B | | | 6.0kb | | 6.0kb | | | | | | 6.0kb | | | |
| UMC89-B | | 5.2kb | | 5.2kb | | | | | | | | 5.2kb | 5.2kb | 5.2kb |
| UMC89-B | | 4.5kb | | 4.5kb | 4.5kb | | | | | | | 4.5kb | 4.5kb | 4.5kb |
| BNL12.30-ERI | | | 3.5kb | | | | | | | | | | | |
| UMC48-H | | | | | 4.7kb | | | | 4.7kb | | 4.7kb | | | |
| UMC48-H | | | | 3.5kb | 3.5kb | | | | 3.5kb | 3.5kb | | | | 3.5kb |
| UMC48-H | | 2.2kb | | | | | | | | | | | | |
| UMC53-ERI | | | 3.8kb | 3.8kb | 3.8kb | | | | 3.8kb | 3.8kb | 3.8kb | | | 3.8kb |
| UMC53-ERI | | 3.0kb | | | | | | | | | | | | |
| npi268-B | | 6.4kb | 6.4kb | 6.4kb | 6.4kb | | | | 6.4kb | 6.4kb | 6.4kb | 6.4kb | 6.4kb | 6.4kb |
| UMC3-ERI | | 2.5kb | | 2.5kb | 2.5kb | | | | 2.5kb | 2.5kb | | 2.5kb | 2.5kb | 2.5kb |
| UMC3-ERI | | 2.0kb | 2.Okb | | 2.0kb | | | | | | | | | |
| | | | | | | | | | | | | | | |

| **Chromosome 9** | | | | | | | | | | | | | | |
|---|---|---|---|---|---|---|---|---|---|---|---|---|---|---|
| phi10005-ERI | | 15.0kb | 15.0kb | 15.0kb | 15.0kb | | | | 15.0kb | 15.0kb | 15.0kb | 15.0kb | 15.0kb | |
| UMC113-ERI | | 5.9kb | | | | | | | | | | | | |
| UMC113-ERI | | | | 5.4kb | 5.4kb | | | | | | | | | |
| UMC113-B | | | | 12.8kb | | | | | | | | | | |
| UMC113-B | | | 11.8kb | | | | | | | | | | | |
| UMC113-B | | 10.5kb | 10.5kb | 10.5kb. | 10.5kb | | | | 10.5kb | 10.5kb | 10.5kb | 10.5kb | 10.5kb | 10.5kb |
| UMC192-H | | 11.4kb | 11.4kb | 11.4kb | 11.4kb | | | | 11.4kb | 11.4kb | 11.4kb | 11.4kb | 11.4kb | 11.4kb |
| UMC192-H | | | 6.4kb | | 6.4kb | | | | | | 6.4kb | | | |
| wx-H | | 21.0kb | | 21.0kb | | | | | | | | | | |
| CSU147-H | | 5.9kb | 5.9kb | | 5.9kb | | | | | | | | | |
| BNL5.10-H | | 6.1kb | 6.1kb | 6.1kb | | | | | | | | | 6.1kb | |
| BNL5.10-H | | 4.4kb | | | 4.4kb | | | | | | | | | |
| UMC114-B | | 15.0kb | | 15.0kb | 15.0kb | | | | | | | | | |
| UMC114-B | | | | 12.6kb | | | | | | | | | 12.6kb | |
| UMC114-B | | 11.5kb | | | 11.5kb | | | | | | | | | |
| UMC114-B | | | | 10.0kb | 10.0kb | | | | 10.0kb | 10.0kb | | 10.0kb | | 10.0kb |
| UMC114-B | | 8.8kb | 8.8kb | | 8.8kb | | | | | | 8.8kb | | | |
| UMC114-B | | | | 7.5kb | 7.5kb | | | | | 7.5kb | | 7.5kb | | |
| UMC114-B | | 6.5kb | 6.5kb | | 6.5kb | | | | | | | | | |
| UMC95-ERI | | 13.3kb | | 13.3kb | | | | 13.3kb | | | | | | |
| UMC95-ERI | | 5.6kb | | 5.6kb | | | | | | | | | | |
| UMC95-H | | | | 7.7kb | | | | | | | 7.7kb | | | |
| UMC95-H | | 4.8kb | 4.8kb | 4.8kb | | | | | 4.8kb | | 4.8kb | | | |
| UMC95-H | | | | 4.1kb | 4.1kb | | | | 4.1kb | 4.1 kb | 4.1kb | 4.1 kb | 4.1kb | 4.1 kb |
| UMC95-B | | | | 15.0kb | 15.0kb | | | | | | | | | |
| UMC95-B | | | | 9.0kb | 9.0kb | | | | | | | | | |
| CSU61-ERI | | 8.1kb | 8.1kb | 8.1kb | 8.1 kb | | | | 8.1kb | 8.1kb | 8.1kb | | 8.1kb | 8.1kb |
| CSU61-ERI | | 4.8kb | | 4.8kb | | | | | | | | 4.8kb | | 4.8kb |
| BNL7.57-ERI | | | | | | | | | | | | | | |
| BNL7.57-B | | | | | | | | | | | | | | |
| BNL7.57-B | | | | | | | | | | | | | | |
| BNL7.57-B | | | | | | | | | | | | | | |
| BNL7.57-B | | | | | | | | | | | | | | |
| CSU54-ERI | | 14.7kb | 14.7kb | | 14.7kb | | | | | | 14.7kb | | | |
| CSU54-ERI | | | | 12.6kb | | | | | | 12.6kb | | 12.6kb | 12.6kb | 12.6kb |
| BNL7.57-ERI | | 1.0kb | | 1.0kb | | | | | | | | | | |
| BNL7.57-B | | | 11.6kb | | | | | | | | | | | |
| BNL7.57-B | | | 5.9kb | | 5.9kb | | | | 5.9kb | 5.9kb | 5.9kb | | | |
| BNL7.57-B | | 5.5kb | | | | | | | | | | | | |
| BNL7.57-B | | 1.3kb | | | | | | | | | | | | |
| npi97-H | | 3.9kb | | 3.9kb | | | | | 3.9kb | 3.9kb | 3.9kb | 3.9kb | | |
| | | | | | | | | | | | | | | |

| **Chromosome 10** | | | | | | | | | | | | | | |
|---|---|---|---|---|---|---|---|---|---|---|---|---|---|---|
| phi20075-ERI | | 7.1 kb | 7.1kb | | 7.1 kb | | | | | | 7.1kb | | | |
| npi285-ERI | | 15.3kb | | 15.3kb | | | | | | | | | | |
| npi285-ERI | | 12.4kb | | 12.4kb | | | | | | | | 12.4kb | 12.4kb | |
| npi285-ERI | | 9.4kb | | | | | | | | | | | | |
| npi285-ERI | | | 6.0kb | | 6.0kb | | | | | | 6.0kb | | | |
| KSU5-ERI | | 9.8kb | 9.8kb | | 9.8kb | | | | | | | | | |
| KSU5-ERI | | | 7.6kb | | | | | | | | | | | |
| KSU5-ERI | | | 6.1kb | | | | | | | | | | | |
| KSU5-ERI | | | 3.8kb | | | | | | | | | | | |
| KSU5-ERI | | 3.5kb | | 3.5kb | | | | | | | | 3.5kb | 3.5kb | 3.5kb |
| UMC130-ERI | | | 13.5kb | | 13.5kb | | | | | | | | | |
| UMC130-ERI | | 7.0kb | | 7.0kb | 7.0kb | | | | 7.0kb | 7.0kb | 7.0kb | 7.0kb | 7.0kb | 7.0kb |
| UMC130-H | | 4.8kb | | 4.8kb | 4.8kb | | | | 4.8kb | 4.8kb | 4.8kb | 4.8kb | 4.8kb | 4.8kb |
| UMC130-H | | 3.2kb | | 3.2kb | 3.2kb | | | | | | 3.2kb | | | |
| UMC130-B | | 3.2kb | | | | | | | | | | | | |
| UMC152.H | | 12.4kb | | | | | | | | | | | | |
| UMC152-H | | 7.1 kb | | 7.1 kb | | | | | | | | 7.1 kb | 7.1 kb | |
| UMC152-H | | | 5.6kb | 5.6kb | 5.6kb | | | | 5.6kb | 5.6kb | 5.6kb | | | 5.6kb |
| UMC64-H | | 3.3kb | | 3.3kb | | | | | | | | | | |
| phi06005 | | 12.8kb | | 12.8kb | 12.8kb | | | | 12.8kb | 12.8kb | | 12.8kb | 12.8kb | 12.8kb |
| UMC163-H | | | | 12.0kb | 12.0kb | | | | | | | | | |
| UMC163-H | | 7.0kb | | | 7.0kb | | | | | | 7.0kb | | | |
| UMC163-H | | | 4.8kb | 4.8kb | 4.8kb | | | | 4.8kb | 4.8kb | 4.8kb | 4.8kb | 4.8kb | |
| UMC44-H | | | 9.8kb | | | | | | | | | | | |
| UMC44-H | | | | 8.7kb | 8.7kb | | | | | | | | 8.7kb | 8.7kb |
| UMC44-H | | 7.2kb | 7.2kb | | | | | | | | | | | |
| UMC44-H | | | | 5.5kb | 5.5kb | | | | 5.5kb | 5.5kb | | 5.5kb | 5.5kb | 5.5kb |
| UMC44-H | | 4.0kb | | | | | | | | | | | | |
| BNL10.13-H | | 10.8kb | 10.8kb | 10.8kb | 10.8kb | | | | | | 10.8kb | | | |
| npi306-H | | | | | 7.0kb | | | | | | | | | |
| | | | | | | | | | | | | | | |

| **Mitochondria** | | | | | | | | | | | | | | |
|---|---|---|---|---|---|---|---|---|---|---|---|---|---|---|
| pmt1-H | | | | | 2.3kb | | | | | | | | | |
| pmt2-H | | 8.0kb | 8.0kb | 8.0kb | 8.0kb | | | | | | | | | 8.0kb |
| pmt2-H | | | 4.2kb | | | | | | | | | | | |
| pmt2-H | | 2.8kb | 2.8kb | 2.8kb | 2.8kb | | | | | | 2.8kb | 2.8kb | 2.8kb | |
| pmt2-H | | 2.1kb | | | | | | | | | | | | |
| pmt5-H | | 12.3kb | 12.3kb | 12.3kb | 12.3kb | | | | | | | | | |
| pmt5-H | | | 8.1kb | | | | | | | | | | | |
| pmt5-H | | | 3.2kb | | 3.2kb | | | | | | | | | |
| pmt5-H | | 2.5kb | | 2.5kb | 2.5kb | | | | | | | | | 2.5kb |
| | | | | | | | | | | | | | | |

| **Locus Unknown** | | | | | | | | | | | | | | |
|---|---|---|---|---|---|---|---|---|---|---|---|---|---|---|
| tda16-H | | | | | 4.3kb | | | | | | | | | |
| tda17-H tda37-B | | 7.0kb 8.2kb | 7.0kb | 7.0kb | | | | | 7.0kb | 7.0kb | 7.0kb | 7.0kb | 7.0kb | 7.0kb |
| tda37-B | | 6.5kb | | | | | | | | | | | | |
| tda48-H | | | | 8.2kb | 8.2kb | | | | | | | | | |
| tda53-H | | 3.8kb | | | | | | | | | | | | |
| tda53-H | | 2.2kb | 2.2kb | 2.2kb | 2.2kb | | | | | 2.2kb | 2.2kb | 2.2kb | 2.2kb | 2.2kb |
| | | | | | | | | | | | | | | |

**Table 5. Novel Tripsacum Alleles in Tripsacum -diploperennis Hybrids and (Maize X Tripsacum -diploperennis)**

| ***Probe*/*Enzyme*** | | ***Tripsacum* -diploperennis Hybrids** | | | | | **Maize X *Tripsacum* -diploperennis** | | | | | | | |
|---|---|---|---|---|---|---|---|---|---|---|---|---|---|---|
| | | | | | | | | | | | | | | |

| **Chromosome 2** | | **Sun Dance** | **20A** | **Tripsacorn** | **Sun Star** | | **64SS** | **64TC** | **2019** | **3024** | **3028** | **3125** | **4126** | **TC64** |
|---|---|---|---|---|---|---|---|---|---|---|---|---|---|---|
| UMC53-ERV | | 8.4kb | | 8.4kb | 8.4kb | | | | | | | 8.4kb | 8.4kb | |
| | | | | | | | | | | | | | | |
| **Chromosome 3** | | | | | | | | | | | | | | |
| UMC50-B | | 7.8kb | 7.8kb | | | | | | | | | | | |
| UMC50-B | | 5.8kb | | 5.8kb | 5.8kb | | | | 5.8kb | 5.8kb | | 5.8kb | 5.8kb | 5.8kb |
| UMC42-H | | 7.6kb | | | 7.6kb | | | | | | | | | |
| | | | | | | | | | | | | | | |

| **Chromosome 4** | | | | | | | | | | | | | | |
|---|---|---|---|---|---|---|---|---|---|---|---|---|---|---|
| phi20725-ERI | | 9.7kb | | | | | | | 9.7kb | | 9.7kb | | | |
| npi386-H | | | 12.6kb | 12.6kb | 12.6kb | | | | 12.6kb | 12.6kb | | | 12.6kb | 12.6kb |
| UMC42-H | | 7.6kb | | | 7.6kb | | | | | | | | | |
| tda62-B | | 4.8kb | 4.8kb | | | | | | | 4.8kb | 4.8kb | 4.8kb | 4.8kb | |
| | | | | | | | | | | | | | | |

| **Chromosome 5** | | | | | | | | | | | | | | |
|---|---|---|---|---|---|---|---|---|---|---|---|---|---|---|
| UMC9O-H | | | 7.8kb | 7.8kb | 7.8kb | | | | 7.8kb | 7.8kb | 7.8kb | 7.8kb | | 7.8kb |
| UMC27-H | | 8.3kb | | 8.3kb | 8.3kb | | | | 8.3kb | 8.3kb | 8.3kb | 8.3kb | 8.3kb | 8.3kb |
| | | | | | | | | | | | | | | |

| **Chromosome 6** | | | | | | | | | | | | | | |
|---|---|---|---|---|---|---|---|---|---|---|---|---|---|---|
| NPI393-ERI | | 7.0kb | 7.0kb | | | | | | | | | | | |
| UMC28-B | | | | 10.0kb | | | | | | | | | 10.0kb | |
| | | | | | | | | | | | | | | |

| **Chromosome 7** | | | | | | | | | | | | | | |
|---|---|---|---|---|---|---|---|---|---|---|---|---|---|---|
| asg8-H | | | | | 8.7kb | | | | | | | | | |
| UMC110-B | | 3.9kb | | | | | | | | | 3.9kb | 3.9kb | 3.9kb | |
| UMC80-H | | 10.6kb | 10.6kb | 10.6kb | 10.6kb | | | | 10.6kb | 10.6kb | 10.6kb | 10.6kb | 10.6kb | 10.6kb |
| UMC80-H | | 8.2kb | 8.2kb | 8.2kb | 8.2kb | | | | | | | | 8.2kb | |
| BNL6.06-ERI | | 1.9kb | | 1.9kb | | | | | | | | | | |
| | | | | | | | | | | | | | | |

| **Chromosome 8** | | | | | | | | | | | | | | |
|---|---|---|---|---|---|---|---|---|---|---|---|---|---|---|
| UMC48-H | | | 6.2kb | | 6.2kb | | | | | | 6.2kb | | | |
| UMC53-ERV | | 8.4KB | | 8.4KB | 8.4KB | | | | | | | 8.4KB | 8.4KB | |
| | | | | | | | | | | | | | | |
| | | | | | | | | | | | | | | |

| **Chromosome 10** | | **Sun Dance** | **20A** | **Tripsacorn** | **Sup Star** | | **64SS** | **64TC** | **2019** | **3024** | **3028** | **3125** | **4126** | **TC64** |
|---|---|---|---|---|---|---|---|---|---|---|---|---|---|---|
| UMC163-H | | 2.6kb | | | | | | | | | 2.6kb | | | |
| | | | | | | | | | | | | | | |
| **Mitochondria** | | | | | | | | | | | | | | |
| pmt5-H | | 3.6kb | 3.6kb | 3.6kb | 3.6kb | | | | 3.6kb | 3.6kb | 3.6kb | 3.6kb | 3.6kb | 3.6kb |
| | | | | | | | | | | | | | | |
| **Locus Unknown** | | | | | | | | | | | | | | |
| tda168-ERI | | 3.6kb | | | 3.6kb | | | | | | | | | |

### DEPOSIT OF SEEDS

A sample comprising at least 2500 seeds derived from crosses between *Tripsacum dactyloides* and Zea *diploperennis* as described herein were deposited with American Type Culture Collection, 12301 Parklawn Drive, Rockville, Maryland 20852 on August 28, 1992. The accession number is ATCC75297.

The present invention is not limited in scope by the seeds deposited, since the deposited embodiments are intended as illustrations of the invention and any seeds, cell lines, plant parts, plants derived from tissue culture or seeds which are functionally equivalent are within the scope of this invention.

## Claims

1. A method of screening a plant to determine whether the plant is a cross between *Tripsacum* and perennial teosinte, said method comprising the following steps:
(a) crossing a *Tripsacum* female parent with a perennial teosinte pollen parent to produce seed, or crossing a perennial teosinte female parent with a *Tripsacum* pollen donor to produce seed; then
(b) harvesting the seed produced in (a); then
(c) growing plants from the seed harvested in (b); then
(d) isolating the total genomic DNA from the plants in (c); then
(e) digesting said genomic DNA with one to four of the restriction enzymes selected from the group consisting of *Eco*RI*, Eco*RV*, Hind*III and *Bam*HI*;* then
(f) probing said digested genomic DNA with one or more DNA markers selected from the group consisting of the maize nuclear DNA probes, maize mitochondrial DNA probes, and *Tripsacum* DNA probes recited below; and then
(g) determining the presence of one or more of the following restriction fragments of the following fragment sizes, wherein said restriction fragments are **characterized by** the following molecular marker-restriction enzyme associations and the associated fragment sizes selected from the group consisting of
BNL5.62, *Eco*RI*,* 10.3 kb; npi97, *Hind*III*,* 3.9 kb; UMC157, *Eco*RI*,* 6.5 kb and 3.3 kb; UMC157, *Hind*III*,* 5.5 kb; UMC157, *Bam*HI*,* 14.0 kb, 8.6 kb and 4.5 kb; UMC11, *Bam*HI, 7.0 kb; CSU3, *Bam*HI, 10.0 kb, 7.6 kb and 3.5 kb; UMC67, *Eco*RI*,* 19.2 kb; UMC67, *Bam*HI 13.4 kb and 1.6 kb; CSU92, *Bam*HI*,* 13.3 kb and 7.5 kb; asg62, *Bam*HI*,* 12.7 kb, 9.7 kb and 6.6 kb; UMC58, *Hind*III*,* 3.3 kb; CSU164, *Eco*RI, 9.0 kb and 7.0 kb; UMC128, *Hind*III*,* 6.0 kb; UMC107, *Eco*RI*,* 6.3 kb and 5.3 kb; UMC107, *Hind*III*,* 19.2 kb; UMC140, *Eco*RI*,* 5.0 kb; UMC140, *Hind*III*,* 6.5 kb; UMC107, *Eco*RI, 6.3 kb; adh1, *Hind*III, 9.4 kb; adh1, *Bam*HI, 9.4 kb; UMC161. *Hind*III, 3.3 kb; BNL8.29, *Hind*III, 8.3 kb; UMC53, *Eco*RI, 9.4 kb, 3.8 kb and 3.0 kb; UMC53, *Eco*RV, 8.4 kb, 3.8 kb and 3.0 kb; UMC6, *Eco*RI, 3.8 kb; UMC6, *Hind*III 9.4 kb; UMC6, *Bam*HI, 13.2 kb, 12.7 kb, and 7.0 kb; UMC61, *Hind*III, 3.4 and 2.8 kb; UMC34, *Eco*RI, 7.5 kb and 5.4 kb; UMC34, *Hind*III, 8.8 kb, 6.5 kb and 5.8 kb; UMC135. *Hind*III, 11.6 kb and 10.8 kb; UMC131, *Eco*RI, 10.6 kb, 5.8 kb and 4.3 kb; UMC55, *Eco*RI, 3.9 kb; UMC55, *Hind*III, 4.3 kb; UMC5, *Eco*RI, 5.4 kb; UMC5, *Hind*III, 6.5 kb; UMC49, *Bam*HI, 8.2kb, 6.0 kb, 4.2 kb, and 3.2 kb; UMC36, *Bam*HI, 4.2 kb; UMC32, *Hind*III 6.7 kb; asg24, *Hind*III, 7.2 kb and 6.4 kb; UMC121, *Eco*RI, 3.7 kb and 3.2 kb; BNL8.35, *Hind*III*,* 9.9 kb and 8.7 kb; UMC50 *Bam*HI*,* 7.8 kb, 6.8 kb, 5.8 kb and 3.8 kb; UMC42. *Hind*III*,* 10.4 kb, 8.9 kb, 7.6 kb, 3.7 kb and 3.0 kb; npi247, EcoRI, 8.0 kb; npi247 *Hind*III 3.0 kb; UMC10. *Hind*III*,* 5.9 kb and 3.0 kb; UMC10. EcoRI, 6.5 kb and 5.5 kb; UMC102. *Eco*RI*,* 2.7 kb; BNL6.06 *Eco*RI*,* 6.8 kb; BNL5.37, *Hind*III*,* 10.3 kb, 5.8 kb and 3.5 kb; npi296. *Eco*RI*,* 7.9 kb; UMC3. *Eco*RI 2.5 kb and 2.0 kb; npi212. *Hind*III*,* 4.3 kb; npi212, BamHI, 5.4 kb; UMC39 EcoRI, 12.2 kb, 9.2kb, 7.8 kb and 7.1 kb; UMC63. *Hind*III*,* 9.5 kb and 4.3kb; UMC96. *Hind*III*,* 11.8 kb, 6.4 kb and 5.5 kb; UMC96 *Bam*HI, 7.5 kb; UMC2, *Eco*RI, 11.8 kb, 10.4 kb and 8.0 kb; CSU25, *Hind*III*,* 4.5 and 4.3 kb; agrr115, *Eco*RI*.* 8.0 kb and 5.4 kb; agrr115, *Bam*HI*,* 5.4 kb and 3.5 kb; phi20725, *Eco*RI*,* 10.3 kb, 9.7 kb and 7.2 kb; phi20725. *Hind*III*,* 1.5 kb; UMC31. *Eco*RI*,* 5.8 kb; UMC31, *Bam*HI 6.5 kb; BNL5.46. *Hind*III*,* 13.7 kb, 10.5 kb, 9.7 kb and 5.1 kb; npi386, *Hind*III*,* 12.6 kb, 9.3 kb and 8.2 kb; tda62, *Bam*HI*,* 5.5 kb and 4.8 kb; BNL5.71, *Eco*RV*,* 11.3 kb, 6.8 kb, and 5.7 kb; BNL5.71, *Bam*HI*,* 11.3 kb, 6.8 kb and 5.7 kb; UMC156, *Hind*III, 3.0 kb; UMC66, *Eco*RI, 10.5 kb and 6.5 kb; UMC66, *Bam*HI, 3.7 kb; UMC19, *Bam*HI, 12.3 kb; UMC104, *Hind*III, 12.4 kb, 11.6 kb and 7.5 kb; UMC104, *Bam*HI, 9.4 kb; UMC133, *Hind*III, 10.6 kb, 9.9 kb, 9.2 kb and 7.7 kb; UMC52, *Bam*HI, 8.7 kb, 6.9 kb, 3.8 kb, 3.0 kb and 2.0 kb; BNL15.07, *Hind*III, 2.9 kb and 2.7 kb; npi409, *Eco*RI, 9.4 kb; npi409, *Hind*III*,* 10.4 kb, 9.0 kb and 3.9 kb; UMC147, *Hind*III*,* 16.3 kb, 3.8 kb and 2.4 kb; UMC90, *Hind*III, 7.8 kb, 2.8 kb and 2.5 kb; UMC90, *Bam*HI, 9.0 kb; UMC27, *Hind*III, 8.3 and 4.5 kb; tda37, *Bam*HI, 8.2 kb and 6.5 kb; UMC43, *Bam*HI, 9.7 kb, 7.3 kb and 5.7 kb; UMC40, *Bam*HI, 7.2 kb, 4.3 kb and 4.0 kb; BNL7.71. *Hind*III, 10.6 kb; tda62, *Bam*HI, 5.5 kb; UMC68, *Hind*III, 6.0 kb; phi10017, *Bam*HI, 15.1 kb and 9.5 kb; tda50, *Bam*HI, 8.5 kb; npi373, *Hind*III, 6.5 kb, 5.6kb and 3.0 kb; tda204, *Bam*HI*,* 4.0 kb; npi393, *Eco*RI, 12.1 kb, 8.5 kb, 7.0 kb and 5.6 kb; UMC65, *Hind*III*,* 2.9 kb; UMC46, *Eco*RI, 6.5 kb and 5.6 kb; asg7, *Hind*III, 6.3 kb; UMC28, HindIII, 15.8 kb and 11.9 kb; UMC28, *Bam*HI, 10.0 kb, 7.6 kb and 6.6 kb; UMC134, *Bam*HI, 4.7 kb; UMC134, *Hind*III, 7.5 kb; asg8, *Hind*III, 10.8 kb, 8,7 kb and 8.4 kb; 02, *Eco*RI, 9.4 kb; BNL15.40, *Hind*III, 5.8 kb; UMC116, *Eco*RI, 9.5 kb; UMC110,*Bam*HI, 10.6 kb, 4.9 kb and 3.9 kb; BNL8.32. *Hind*III, 8.9 kb, 7.4 kb and 7.1 kb; BNL14.07, *Eco*RI, 6.4 kb; UMC80, *Hind*III, 10.6 kb. 8.2 kb and 2.4 kb; BNL16.06, *Eco*RI, 6.8 kb and 1.9 kb; phi20020, *Hind*III,7.8 kb and 6.6kb; npi114, *Hind*III, 100 kb, 8.8kb and 6.3 kb; BNL9.11, *Hind*III, 3.4 kb; UMC103, *Hind*III, 6.9 kb and 5.7 kb; UMC124, *Hind*III, 8.0 and 7.0; UMC124, BamHI, 6.6 kb and 2.6 kb; UMC120, *Hind*III, 3.2 kb, 2.3 kb and 1.4 kb; UMC89, *Eco*RI, 7.3 kb; UMC89, *Hind*III, 7.3 kb; UMC89, *Bam*HI, 9.5 kb, 6.0 kb, 5.2 kb and 4.5 kb; BNL12.30, *Eco*RI, 3.5 kb; UMC48, *Hind*III*,* 6.2 kb, 5.3 kb, 4.7 kb, 3.5 kb and 2.2 kb; npi268, *Bam*HI, 6.4 kb; phi10005. EcoRI, 15.0 kb; UMC113, *Eco*RI, 5.9 kb and 5.4 kb; UMC113, *Bam*HI, 12.8 kb, 11.8 kb and 10.5 kb; UMC192. *Hind*III, 11.4 kb and 6.4 kb; wx (waxy), *Hind*III, 21.0 kb; CSU147, *Hind*III 5.9 kb; BNL.5.10, *Hind*III, 6.1 kb and 4.4 kb; UMC114, *Bam*HI, 12.6 kb, 11.5 kb, 10.0 kb, 8.8 kb, 7.5 kb and 6.5 kb; UMC95, *Eco*RI, 5.6 kb; UMC95. *Hind*III, 7.7 kb, 4.8 kb and 4.1 kb; UMC95, *Bam*HI. 15.0 kb and 9.0 kb; CSU61. *Eco*RI, 8.1 kb and 4.8 kb; BNL7.57. *Eco*RI, 1.0 kb; BNL7.57 *Bam*HI*,* 11.6 kb, 5.9 kb and 1.3 kb; CSU54, *Eco*RI*,* 14.7 kb and 12.6 kb; phi20075, *Eco*RI, 7.1 kb; npi285, *Eco*RI*,* 12.4 kb, 9.4 kb and 6.0 kb; KSU5, *Eco*RI*,* 9.8 kb, 7.6 kb, 6.1 kb, 3.8 kb and 3.5 kb; UMC130, *Eco*RI, 13.5 kb and 7.0 kb; UMC130, *Hind*III, 4.8 kb and 3.2 kb; UMC130. *Bam*HI, 3.2 kb; UMC152, *Hind*III, 12.4, 7.1 kb and 5.6 kb; UMC64 *Hind*III, 3.3 kb; phi06005, *Eco*RI, 12.8 kb; UMC163, *Hind*III, 7.0 kb, 4.8 kb and 2.6 kb; UMC44, *Hind*III, 9.8 kb, 8.7 kb, 7.2 kb, 5.5 kb and 4.0 kb; BNL10.13, *Hind*III, 10.8 kb; npi306. *Hind*III, 7.0 kb; pmt1, *Hind*III, 2.3 kb; pmt2, *Hind*III, 8.0 kb, 4.2 kb, 2.8 kb and 2.1 kb; pmt5. *Hind*III, 12.3 kb, 8.1 kb, 3.6 kb, 3.2 kb and 2.5 kb; tda48, *Hind*III, 8.2 kb; tda53, *Hind*III, 3.8 kb and 2.2 kb; tda 168, *Eco*RI, 3.6 kb; tda 16, *Hind*III*, 4.3* kb; and tda17, *Hind*III*,* 7.0 kb.

2. A method of producing hybrid maize seed that contains one or more restriction fragments according to claim 1 thereof, comprising the steps of:
(a) crossing a *Tripsacum* female parent with a perennial teosinte male parent to produce *(Tripsacum* X perennial teosinte) hybrid seed or a perennial teosinte female parent with a *Tripsacum* pollen donor plant to produce hybrid seed;
(b) growing a hybrid plant from said seed to maturity;
(c) screening said hybrid plant to determine whether the plant is a cross between *Tripsacum* and perennial teosinte using the method of claim 1;
(d) crossing said *(Tripsacum* X perennial teosinte) or (perennial teosinte X *Tripsacum)* hybrid plant with maize to produce seed; and
(e) harvesting the seed produced in (d).

3. A method of producing a hybrid maize plant that contains one or more restriction fragments according to claim 1, which method comprises:
(a) producing hybrid maize seed using a method according to claim 2; and
(b) growing the hybrid maize seed to give a hybrid maize plant.

4. A method for producing a derivative, variant, mutant, modification, cellular component, molecular component, pollen or tissue culture from a hybrid maize plant, which method comprises:
(a) producing hybrid maize seed using a method according to claim 2;
(b) growing the hybrid maize seed to give a hybrid maize plant; and
(c) obtaining a derivative, variant, mutant, modification, cellular component, molecular component, pollen or tissue culture from the hybrid maize plant.

## Patentansprüche

1. Screeningverfahren einer Pflanze zur Bestimmung, ob die Pflanze eine Kreuzung zwischen Tripsacum und ausdauernder Teosinte ist, wobei das Verfahren die folgenden Schritte umfaßt:
(a) Kreuzung eines weiblichen Tripsacum-Elternteils mit einem ausdauernden Teosinte-Pollenelternteil zur Erzeugung von Samen oder Kreuzung eines weiblichen ausdauernden Teosinte-Elternteils mit einem Tripsacum-Pollendonor zur Erzeugung eines Samens; dann
(b) Ernte des in (a) erzeugten Samens; dann
(c) Züchten von Pflanzen aus dem in (b) geernteten Samen; dann
(d) Isolation der gesamten genomischen DNA von den Pflanzen in (c); dann
(e) Verdau der genomischen DNA mit ein bis vier der Restriktionsenzyme, ausgewählt aus der Gruppe bestehend aus EcoRI, EcoRV, HindIII und BamHI; dann
(f) Durchsuchen der verdauten genomischen DNA mit ein oder mehr DNA-Markern als Sonden, ausgewählt aus der Gruppe bestehend aus den nukleären Mais-DNA-Sonden, Mitochondrien-Mais-DNA-Sonden und Tripsacum DNA-Sonden wie unten angegeben; und dann
(g) Bestimmung der Gegenwart von ein oder mehr der folgenden Restriktionsfragmente der folgenden Fragmentgrößen, wobei die Restriktionsfragmente durch die folgenden Molekularmarker-Restriktionsenzym-Assoziationen und die assoziierten Fragmentgrößen **gekennzeichnet** sind, die aus der Gruppe ausgewählt werden bestehend aus:
BNL5.62, EcoRI, 10,3 kb; npi97, HindIII, 3,9 kb; UMC157, EcoRI, 6,5 kb und 3,3 kb; UMC157, HindIII, 5,5 kb; UMC157, BamHI, 14,0 kb, 8,6 kb und 4,5 kb; UMC11, BamHI, 7,0 kb; CSU3, BamHI, 10,0 kb, 7,6 kb und 3,5 kb; UMC67, EcoRI, 19,2 kb; UMC67, BamHI, 13,4 kb und 1,6 kb; CSU92, BamHI, 13,3 kb und 7,5 kb; asg62, BamHI, 12,7 kb, 9,7 kb und 6,6 kb; UMC58, HindIII, 3,3 kb; CSU164, EcoRI, 9,0 kb und 7,0 kb; UMC128, HindIII, 6,0 kb; UMC107, EcoRI, 6,3 kb und 5,3 kb; UMC107, HindIII, 19,2 kb; UMC140, EcoRI, 5,0 kb; UMC140, HindIII, 6,5 kb; UMC107, EcoRI, 6,3 kb; adh1, HindIII, 9,4 kb; adh1, BamHI, 9,4 kb; UMC161, HindIII, 3,3 kb; BNL8.29, HindIII, 8,3 kb; UMC53, EcoRI, 9,4 kb, 3,8 kb und 3,0 kb; UMC53, EcoRV, 8,4 kb, 3,8 kb und 3,0 kb; UMC6, EcoRI, 3,8 kb; UMC6, HindIII, 9,4 kb; UMC6, BamHI, 13,2 kb, 12,7 kb und 7,0 kb; UMC61, HindIII, 3,4 kb und 2,8 kb; UMC34, EcoRI, 7,5 kb und 5,4 kb; UMC34, HindIII, 8,8 kb, 6,5 kb und 5,8 kb; UMC135, HindIII, 11,6 kb und 10,8 kb; UMC131, EcoRI, 10,6 kb, 5,8 kb und 4,3 kb; UMC55, EcoRI, 3,9 kb; UMC55, HindIII, 4,3 kb; UMC5, EcoRI, 5,4 kb; UMC5, HindIII, 6,5 kb; UMC49, BamHI, 8,2 kb, 6,0 kb, 4,2 kb und 3,2 kb; UMC36, BamHI, 4,2 kb; UMC32, HindIII, 6,7 kb; asg24, HindIII, 7,2 kb und 6,4 kb; UMC121, EcoRI, 3,7 kb und 3,2 kb; BNL8.35, HindIII, 9,9 kb und 8,7 kb; UMC50, BamHI, 7,8 kb, 6,8 kb, 5,8 kb und 3,8 kb; UMC42, HindIII, 10,4 kb, 8,9 kb, 7,6 kb, 3,7 kb und 3,0 kb; npi247, EcoRI, 8,0 kb; npi247, HindIII, 3,0 kb; UMC10, HindIII, 5,9 kb und 3,0 kb; UMC10, EcoRI, 6,5 kb und 5,5 kb; UMC102, EcoRI, 2,7 kb; BNL6.06, EcoRI, 6,8 kb; BNL5.37, HindIII, 10,3 kb, 5,8 kb und 3,5 kb; npi296, EcoRI, 7,9 kb; UMC3, EcoRI, 2,5 kb und 2,0 kb; npi212, HindIII, 4,3 kb; npi212, BamHI, 5,4 kb; UMC39, EcoRI, 12,2 kb, 9,2 kb, 7,8 kb und 7,1 kb; UMC63, HindIII, 9,5 kb und 4,3 kb; UMC96, HindIII, 11,8 kb, 6,4 kb und 5,5 kb; UMC96, BamHI, 7,5 kb, UMC2, EcoRI, 11,8 kb, 10,4 kb und 8,0 kb; CSU25, HindIII, 4,5 kb und 4,3 kb; agrr115, EcoRI, 8,0 kb und 5,4 kb; agrr115, BamHI, 5,4 kb und 3,5 kb; phi20725, EcoRI, 10,3 kb, 9,7 kb und 7,2 kb; phi20725, HindIII, 1,5 kb; UMC31, EcoRI, 5,8 kb; UMC31, BamHI, 6,5 kb; BNL5.46, HindIII, 13,7 kb, 10,5 kb, 9,7 kb und 5,1 kb; npi386, HindIII, 12,6 kb, 9,3 kb und 8,2 kb; tda62, BamHI, 5,5 kb und 4,8 kb; BNL5.71, EcoRV, 11,3 kb, 6,8 kb und 5,7 kb; BNL5.71, BamHI, 11,3 kb, 6,8 kb und 5,7 kb; UMC156, HindIII, 3,0 kb; UMC66, EcoRI, 10,5 kb und 6,5 kb; UMC66, BamHI, 3,7 kb; UMC19, BamHI, 12,3 kb; UMC104, HindIII, 12,4 kb, 11,6 kb und 7,5 kb; UMC104, BamHI, 9,4 kb; UMC133, HindIII, 10,6 kb, 9,9 kb, 9,2 kb und 7,7 kb; UMC52, BamHI, 8,7 kb, 6,9 kb, 3,8 kb, 3,0 kb und 2,0 kb; BNL15.07, HindIII, 2,9 kb und 2,7 kb; npi409, EcoRI, 9,4 kb; npi409, HindIII, 10,4 kb, 9,0 kb und 3,9 kb; UMC147, HindIII, 16,3 kb, 3,8 kb und 2,4 kb; UMC90, HindIII, 7,8 kb, 2,8 kb und 2,5 kb; UMC90, BamHI, 9,0 kb; UMC27, HindIII, 8,3 kb und 4,5 kb; tda37, BamHI, 8,2 kb und 6,5 kb; UMC43, BamHI, 9,7 kb, 7,3 kb und 5,7 kb; UMC40, BamHI, 7,2 kb, 4,3 kb und 4,0 kb; BNL7.71, HindIII, 10,6 kb; tda62, BamHI, 5,5 kb; UMC68, HindIII, 6,0 kb; phi10017, BamHI, 15,1 kb und 9,5 kb; tda50, BamHI, 8,5 kb; npi373, HindIII, 6,5 kb, 5,6 kb und 3,0 kb; tda204, BamHI, 4,0 kb; npi393, EcoRI, 12,1 kb, 8,5 kb, 7,0 kb und 5,6 kb; UMC65, HindIII, 2,9 kb; UMC46, EcoRI, 6,5 kb und 5,6 kb; asa7, HindIII, 6,3 kb; UMC28, HindIII, 15,8 kb und 11,9 kb; UMC28, BamHI, 10,0 kb, 7,6 kb und 6,6 kb; UMC134, BamHI, 4,7 kb; UMC134, HindIII, 7,5 kb; asg8, HindIII, 10,8 kb, 8,7 kb und 8,4 kb; O2, EcoRI, 9,4 kb; BNL15.40, HindIII, 5,8 kb; UMC116, EcoRI, 9,5 kb; UMC110, BamHI, 10,6 kb, 4,9 kb und 3,9 kb; BNL8.32, HindIII, 8,9 kb, 7,4 kb und 7,1 kb; BNL14.07, EcoRI, 6,4 kb; UMC80, HindIII, 10,6 kb, 8,2 kb und 2,4 kb; BNL16.06, EcoRI, 6,8 kb und 1,9 kb; phi20020, HindIII, 7,8 kb und 6,6 kb; npi114, HindIII, 10,0 kb, 8,8 kb und 6,3 kb; BNL9.11, HindIII, 3,4 kb; UMC103, HindIII, 6,9 kb und 5,7 kb; UMC124, HindIII, 8,0 kb und 7,0 kb; UMC124, BamHI, 6,6 kb und 2,6 kb; UMC120, HindIII, 3,2 kb, 2,3 kb und 1,4 kb; UMC89, EcoRI, 7,3 kb; UMC89, HindIII, 7,3 kb; UMC89, BamHI, 9,5 kb, 6,0 kb, 5,2 kb und 4,5 kb; BNL12.30, EcoRI, 3,5 kb; UMC48, HindIII, 6,2 kb, 5,3 kb, 4,7 kb, 3,5 kb und 2,2 kb; npi268, BamHI, 6,4 kb; phi10005, EcoRI, 15,0 kb; UMC113, EcoRI, 5,9 kb und 5,4 kb; UMC113, BamHI, 12,8 kb, 11,8 kb und 10,5 kb; UMC192, HindIII, 11,4 kb und 6,4 kb; wx (waxy), HindIII, 21,0 kb; CSU147, HindIII, 5,9 kb; BNL5.10, HindIII, 6,1 kb und 4,4 kb; UMC114, BamHI, 12,6 kb, 11,5 kb, 10,0 kb, 8,8 kb, 7,5 kb und 6,5 kb; UMC95, EcoRI, 5,6 kb; UMC95, HindIII, 7,7 kb, 4,8 kb und 4,1 kb; UMC95, BamHI, 15,0 kb und 9,0 kb; CSU61, EcoRI, 8,1 kb und 4,8 kb; BNL7.57, EcoRI, 1,0 kb, BNL7.57, BamHI, 11,6 kb, 5,9 kb und 1,3 kb; CSU54, EcoRI, 14,7 kb und 12,6 kb; phi20075, EcoRI, 7,1 kb; npi285, EcoRI, 12,4 kb, 9,4 kb und 6,0 kb; KSU5, EcoRI, 9,8 kb, 7,6 kb, 6,1 kb, 3,8 kb und 3,5 kb; UMC130, EcoRI, 13,5 kb und 7,0 kb; UMC130, HindIII, 4,8 kb und 3,2 kb; UMC130, BamHI, 3,2 kb; UMC152, HindIII, 12,4 kb, 7,1 kb und 5,6 kb; UMC64, HindIII, 3,3 kb; phi06005, EcoRI, 12,8 kb; UMC163, HindIII, 7,0 kb, 4,8 kb und 2,6 kb; UMC44, HindIII, 9,8 kb, 8,7 kb, 7,2 kb, 5,5 kb und 4,0 kb; BNL10.13, HindIII, 10,8 kb; npi306, HindIII, 7,0 kb; pmt1, HindIII, 2,3 kb; pmt2, HindIII, 8,0 kb, 4,2 kb, 2,8 kb und 2,1 kb; pmt5. HindIII, 12,3 kb, 8,1 kb, 3,6 kb, 3,2 kb und 2,5 kb; tda48, HindIII, 8,2 kb; tda53, HindIII, 3,8 kb und 2,2 kb; tda168, EcoRI, 3,6 kb; tda16, HindIII, 4,3 kb und tda17, HindIII, 7,0 kb.

2. Verfahren zur Erzeugung von Hybridmaissamen, der ein oder mehr Restriktionsfragmente enthält, gemäß Anspruch 1, umfassend die Schritte:
(a) Kreuzung eines weiblichen Tripsacum-Elternteils mit einem männlichen ausdauernden Teosinte-Elternteil zur Erzeugung von (Tripsacum X ausdauernder Teosinte) Hybridsamen oder eines weiblichen ausdauernden Teosinte-Elternteils mit einer Tripsacum-Pollendonorpflanze zur Erzeugung eines Hybridsamens;
(b) Züchten einer Hybridpflanze aus dem Samen bis zur Reife;
(c) Screening der Hybridpflanze zur Bestimmung, ob die Pflanze eine Kreuzung zwischen Tripsacum und ausdauernder Teosinte ist, wobei das Verfahren gemäß Anspruch 1 verwendet wird;
(d) Kreuzen der (Tripsacum X ausdauernder Teosinte) oder (ausdauernder Teosinte X Tripsacum) Hybridpflanze mit Mais zur Erzeugung eines Samens; und
(e) Ernten des in (d) erzeugten Samens.

3. Verfahren zur Erzeugung eines Hybridmaispflanze, die ein oder mehr Restriktionsfragmente enthält, gemäß Anspruch 1, wobei das Verfahren folgendes umfaßt:
(a) Erzeugung eines Hybridmaissamens unter Verwendung eines Verfahrens gemäß Anspruch 2 und
(b) Züchten des Hybridmaissamens zum Erhalt einer Hybridmaispflanze.

4. Verfahren zur Erzeugung eines Derivats, einer Variante, einer Mutante, einer Modifikation, einer zellulären Komponente, einer molekularen Komponente, eines Pollens oder einer Gewebekultur einer Hybridmaispflanze, wobei das Verfahren folgendes umfaßt:
(a) Erzeugung eines Hybridmaissamens unter Verwendung eines Verfahrens gemäß Anspruch 2;
(b) Züchten des Hybridmaissamens zum Erhalt einer Hybridmaispflanze und
(c) Erhalt eines Derivats, einer Variante, einer Mutante, einer Modifikation, einer zellulären Komponente, einer molekularen Komponente, eines Pollens oder einer Gewebekultur aus der Hybridmaispflanze.

## Revendications

1. Procédé d'analyse d'une plante pour déterminer si la plante est le résultat d'un croisement entre *Tripsacum* et le téosinte pérenne, ledit procédé comprenant les étapes suivantes :
(a) croisement d'un parent *Tripsacum* femelle avec un parent consistant en pollen de téosinte pérenne pour la production de graines, ou croisement d'un parent téosinte pérenne femelle avec un donneur de pollen *Tripsacum* pour la production de graines ; puis
(b) récolte des graines produites en (a) ; puis
(c) culture des plants provenant des graines récoltées en (b) ; puis
(d) isolement de l'ADN génomique total à partir des plants en (c) ; puis
(e) digestion dudit ADN génomique par une à quatre des enzymes de restriction choisies dans le groupe constitué par *Eco*RI*, Eco*RV*, Hin*dIII et *Bam*HI ; puis
(f) sondage dudit ADN génomique digéré, à l'aide d'un ou plusieurs marqueurs d'ADN choisis dans le groupe constitué par des sondes d'ADN nucléaire de maïs, des sondes d'ADN mitochondrial de maïs et des sondes d'ADN de *Tripsacum* indiquées ci-dessous ; et ensuite
(g) détermination de la présence d'un ou plusieurs fragments de restriction des tailles de fragment suivantes, lesdits fragments de restriction étant **caractérisés par** les associations marqueur moléculaire-enzyme de restriction suivantes et les tailles suivantes de fragments, choisies dans le groupe constitué par :
BNL5.62, *Eco*RI, 10,3 kb; npi97, *Hin*dIII*,* 3,9 kb; UMC157, *EcoRI,.* 6,5 kb et 3,3 kb ; UMC157, *Hin*dIII, 5,5 kb ; UMC157, *BamHI,* 14,0 kb, 8,6 kb et 4,5 kb ; UMC11, *BamHI,* 7,0 kb ; CSU3, *BamHI,* 10,0 kb, 7,6 kb et 3,5 kb; UMC67, *Eco*RI, 19,2 kb; UMC67, *BamHI* 13,4 kb et 1,6 kb; CSU92, *BamHI,* 13,3 kb et 7,5 kb ; asg62, *BamHI,* 12,7 kb, 9,7 kb et 6,6 kb ; UMC58, *Hin*dIII*,* 3,3 kb ; CSU164, *Eco*RI, 9,0 kb et 7,0 kb; UMC128, *Hin*dIII*,* 6,0 kb ; UMC107, *EcoRI,* 6,3 kb et 5,3 kb ; UMC107, *Hin*dIII*;* 19,2 kb ; UMC140, *EcoRI,* 5,0 kb ; UMC140, *Hin*dIII*,* 6,5 kb ; UMC107, *EcoRI,* 6,3 kb ; adhI, *Hin*dIII*,* 9,4 kb ; adhI, *Bam*HI*,* 9,4 kb ; UMC161, *Hin*dIII*,* 3,3 kb ; BNL8.29, *Hin*dIII, 8,3 kb ; UMC53, *Eco*RI, 9,4 kb, 3,8 kb et 3,0 kb ; UMC53. *Eco*RV, 8,4 kb, 3,8 kb et 3,0 kb ; UMC6, *Eco*RI, 3,8 kb ; UMC6. *Hind*III, 9,4 kb ; UMC6, *Bam*HI, 13,2 kb, 12,7 kb et 7,0 kb ; UMC61, *Hin*dIII, 3,4 et 2,8 kb ; UMC34, *Eco*RI, 7,5 kb et 5,4 kb ; UMC34, *Hin*dIII*,* 8,8 kb, 6,5 kb et 5,8 kb ; UMC135. *Hin*dIII, 11,6 kb et 10,8 kb ; UMC131, *Eco*RI, 10,6 kb, 5,8 kb et 4,3 kb ; UMC55, *Eco*RI*,* 3,9 kb ; UMC55, *Hin*dIII, 4,3 kb ; UMC5, *Eco*RI, 5,4 kb ; UMC5, *Hin*dIII*,* 6,5. kb ; UMC49, *Bam*HI, 8,2kb, 6,0 kb, 4,2 kb, et 3,2 kb ; UMC36, *Bam*HI, 4,2 kb ; UMC32, *Hin*dIII 6,7 kb ; asg24, *Hin*dIII, 7,2 kb et 6,4 kb ; UMC121, *Eco*RI, 3,7 kb et 3,2 kb ; BNL8.35, *Hin*dIII, 9,9 kb et 8,7 kb ; UMC50, *Bam*HI, 7,8 kb, 6,8 kb, 5,8 kb et 3,8 kb ; UMC42, *Hin*dIII, 10,4 kb, 8,9 kb, 7,6 kb, 3,7 kb et 3,0 kb ; npi247, *Eco*RI, 8,0 kb ; npi247, *Hin*dIII, 3,.0 kb ; UMC10, *Hin*dIII, 5,9 kb et 3,0 kb ; UMC10, *Eco*RI, 6,5 kb et 5,5 kb ; UMC102, *Eco*RI*,* 2,7 kb ; BNL6.06, *Eco*RI*,* 6,8 kb ; BNL5.37, *Hin*dIII*,* 10,3 kb, 5,8 kb et 3,5 kb ; npi296, *Eco*RI*,* 7,9 kb ; UMC3, *Eco*RI 2,5 kb et 2,0 kb ; npi212, *Hin*dIII*,* 4,3 kb ; npi212, *Bam*HI*,* 5,4 kb ; UMC39, *Eco*RI*,* 12,2 kb, 9,2kb, 7,8 kb et 7,1 kb ; UMC63, *Hin*dIII*,* 9,5 kb et 4,3 kb ; UMC96, *Hin*dIII*,* 11,8 kb, 6,4 kb et 5,5 kb; UMC96, *Bam*HI*,* 7,5 kb ; UMC2, *Eco*RI*,* 11,8 kb, 10,4 kb et 8,0 kb ; CSU25, *Hin*dIII, 4,5 et 4,3 kb ; agrr115, *Eco*RI, 8,0 kb et 5,4 kb ; agrr115, *Bam*HI*,* 5,4 kb et 3,5 kb; phi20725, *Eco*RI*,* 10,3 kb, 9,7 kb et 7,2 kb; phi20725, *Hin*dIII*,* 1,5 kb ; UMC31, *Eco*RI*,* 5,8 kb ; UMC31, *Bam*HI 6,5 kb ; *BNL5.46, Hin*dIII, 13,7 kb, 10,5 kb, 9,7 kb et 5,1 kb ; npi386, *Hin*dIII, 12,6 kb, 9,3 kb et 8,2 kb ; tda62, *Bam*HI*,* 5,5 kb et 4,8 kb ; BNL5.71, *Eco*RV*,* 11,3 kb, 6,8 kb, et 5,7 kb ; BNL5.71, *Bam*HI*,* 11,3 kb, 6,8 kb et 5,7 kb ; UMC156, *Hin*dIII, 3,0 kb ; UMC66, *EcoRI,* 10,5 kb et 6,5 kb; UMC66, *Bam*HI*,* 3,7 kb; UMC19, *Bam*HI*,* 12,3 kb; UMC104, *Hin*dIII*,* 12,4 kb, 11,6 kb et 7,5 kb, UMC104, *Bam*HI*,* 9,4 kb ; UMC 133, *Hin*dIII, 10,6 kb, 9,9 kb, 9,2 kb et 7,7 kb ; UMC52, *Bam*HI*,* 8,7 kb, 6,9 kb, 3,8 kb, 3,0 kb et 2,0 kb ; BNL15.07, *Hin*dIII*,* 2,9 kb et 2,7 kb ; npi409, *Eco*RI*,* 9,4 kb ; npi409, *Hin*dIII, 10,4 kb, 9,0 kb et 3,9 kb ; UMC147, *Hin*dIII, 16,3 kb, 3,8 kb et 2,4 kb ; UMC90, *Hin*dIII*,* 7,8 kb, 2,8 kb et 2,5 kb ; UMC90, *Bam*HI*,* 9,0 kb ; UMC27, *Hin*dIII*,* 8,3 et 4,5 kb ; tda37, *BamHI,* 8,2 kb et 6,5 kb ; UMC43, *BamHI,* 9,7 kb, 7,3 kb et 5,7 kb ; UMC40, *BamHI,* 7,2 kb, 4,3 kb et 4,0 kb ; BNL7.71, *Hin*dIII*,* 10,6 kb; tda62, *BamHI,* 5,5 kb ; UMC68, *Hin*dIII*,* 6,0 kb ; phi10017, *BamHI,* 15,1 kb et 9,5 kb ; tda50, *BamHI,* 8,5 kb ; npi373, *Hin*dIII*,* 6,5 kb, 5,6 kb et 3,0 kb ; tda204, *BamHI,* 4,0 kb ; npi393, EcoRI, 12,1 kb, 8,5 kb, 7,0 kb et 5,6 kb ; UMC65, *Hin*dIII*;* 2,9 kb ; UMC46, *Eco*RI*,* 6,5 kb et 5,6 kb ; asg7, *Hin*dIII*,* 6,3 kb ; UMC28, *Hin*dIII, 15,8 kb et 11,9 kb ; UMC28, *Bam*HI*,* 10,0 kb, 7,6 kb et 6,6 kb; UMC134, *Bam*HI*,* 4,7 kb ; UMC134, *Hin*dIII*,* 7,5 kb ; asg8, *Hin*dIII, 10,8 kb, 8,7 kb et 8,4 kb ; O2, EcoRI, 9,4 kb; BNL15.40, *Hin*dIII*,* 5,8 kb ; UMC116, *Eco*RI*,* 9,5 kb ; UMC110, *Bam*HI*;* 10,6 kb, 4,9 kb et 3,9 kb ; BNL8.32, *Hin*dIII*,* 8,9 kb, 7,4 kb et 7,1 kb; BNL14.07, *Eco*RI*,* 6,4 kb ; UMC80, *Hin*dIII*,* 10,6 kb, 8,2 kb et 2,4 kb ; BNL16.06, *Eco*RI, 6,8 kb et 1,9 kb ; phi20020, *Hin*dIII, 7,8 kb et 6,6 kb ; npil114, *Hin*dIII*,* 10,0 kb, 8,8 kb et 6,3 kb; BNL9.11, *Hin*dIII*,* 3,4 kb; UMC103, *Hin*dIII, 6,9 kb et 5,7 kb ; UMC124, *Hin*dIII*,* 8,0 et 7.0; UMC124, *Bam*HI*,* 6,6 kb et 2,6 kb ; UMC120, *Hin*dIII*;* 3,2 kb, 2,3 kb et 1,4 kb ; UMC89, *Eco*RI*,* 7,3 kb ; UMC89, *Hin*dIII*,* 7,3 kb ; UMC89, *Bam*HI*,* 9,5 kb, 6,0 kb, 5,2 kb et 4,5 kb ; BNL12.30, *EcoRI,* 3,5 kb ; UMC48, *Hin*dIII*,* 6,2 kb, 5,3 kb, 4,7 kb, 3,5 kb et 2,2kb ; npi268,- *BamHI,* 6,4 kb ; phi10005, *EcoRI,* 15,0 kb; UMC113, EcoRI, 5,9 kb et 5,4 kb ; UMC113, *BamHI;* 12,8 kb, 11,8 kb et 10,5 kb; UMC192, *Hin*dIII*,* 11,4 kb et 6,4 kb ; wx (waxy), *Hin*dIII*,* 21,0 kb ; CSU147, *Hin*dIII 5,9 kb ; BNL5.10, *Hin*dIII*,* 6,1 kb et 4,4 kb ; UMC114, *Bam*HI*.* 12,6 kb, 11,5 kb, 10,0 kb, 8,8 kb, 7,5 kb et 6,5 kb ; UMC95, *EcoRI,* 5,6 kb ; UMC95, *Hin*dIII*,* 7,7 kb, 4,8 kb et 4,1 kb ; UMC95, *BamHI,* 15,0 kb et 9,0 kb ; CSU61, *EcoRI,* 8,1 kb et 4,8 kb ; BNL7.57, *EcoRI,* 1,0 kb ; BNL7.57, *BamHI,* 11,6 kb, 5,9 kb et 1,3 kb ; CSU54, *EcoRI ;* 14,7 kb et 12,6 kb; phi20075, *Eco*RI*,* 7,1 kb; npi285_{.} *Eco*RI*,* 12,4 kb, 9,4 kb et 6,0 kb ; KSU5, *Eco*RI*,* 9,8 kb; 7,6 kb, 6,1 kb, 3,8 kb et 3,.5 kb, UMC130, *Eco*RI, 13,5 kb et 7,0 kb ; UMC130, *Hin*dIII*,* 4,8 kb et 3,2 kb ; UMC130, *Bam*HI*,* 3,2 kb ; UMC152, *Hin*dIII*,* 12,4, 7,1 kb et 5,6 kb ; UMC64, *Hin*dIII *;* 3,3 kb; phi06005, *EcoRI,* 12,8 kb; UMC163, *Hin*dIII, 7,0 kb, 4,8 kb et 2,6 kb; UMC44, *Hin*dIII, 9,8 kb, 8,7 kb, 7,2 kb, 5,5 kb et 4.0 kb ; BNL10.13, *Hin*dIII, 10,8 kb; npi306, *Hin*dIII*,* 7,0 kb ; pmt1, *Hin*dIII, 2,3 kb, pmt2, *Hin*dIII, 8,0 kb, 4,2 kb, 2,8 kb et 2,1 kb ; pmt5, *Hin*dIII, 12,3 kb, 8,1 kb, 3,6 kb, 3,2 kb et 2,5 kb ; tda48, *Hin*dIII, 8,2 kb ; tda53, *Hin*dIII*,* 3,8 kb et 2,2 kb ; tda168, *Eco*RI, 3,6 kb ; tda16, *Hin*dIII, 4,3 kb ; et tda 17, *Hin*dIII, 7,0 kb.

2. Procédé de production d'une graine de maïs hybride qui contient un ou plusieurs fragments de restriction selon la revendication 1, comprenant les étapes suivantes :
(a) croisement d'un parent *Tripsacum* femelle avec un parent téosinte pérenne mâle pour la production d'une graine hybride *(Tripsacum* x téosinte pérenne) ou d'un parent téosinte pérenne femelle avec une plante *Tripsacum* donneuse de pollen, pour la production d'une graine hybride ;
(b) culture d'une plante hybride à partir de ladite graine, jusqu'à la maturité ;
(c) analyse de ladite plante hybride pour déterminer si la plante est issue d'un croisement entre *Tripsacum* et le téosinte pérenne, au moyen du procédé de la revendication 1 ;
(d) croisement de ladite plante hybride *(Tripsacum* × téosinte pérenne) or (téosinte pérenne x *Tripsacum)* avec le maïs pour produire des graines ; et
(e) récolte des graines produites en (d).

3. Procédé de production d'un plant de maïs hybride qui contient un ou plusieurs fragments de restriction selon la revendication 1, lequel procédé comprend :
(a) la production d'une graine de maïs hybride, au moyen d'un procédé selon la revendication 2 ; et
(b) la culture de la graine de maïs hybride pour obtenir un plant de maïs hybride.

4. Procédé pour la production d'un dérivé, d'un variant, d'un mutant, d'une modification, d'un composant cellulaire, d'un composant moléculaire, de pollen ou d'une culture de tissu à partir d'un plant de maïs hybride, lequel procédé comprend:
(a) la production d'une graine de maïs hybride au moyen d'un procédé selon la revendication 2;
(b) la culture de la graine de maïs hybride; pour obtenir un plant de maïs hybride ; et
(c) l'obtention d'un dérivé, d'un variant, d'un mutant, d'une modification, d'un composant cellulaire, d'un composant moléculaire, de pollen ou d'une culture de tissu à partir du plant de maïs hybride.
